(19) Europäisches Patentamt
European Patent Office
Office européen des brevets

(11) EP 2 045 256 A1

(12) # EUROPEAN PATENT APPLICATION
published in accordance with Art. 153(4) EPC

(43) Date of publication:
**08.04.2009 Bulletin 2009/15**

(21) Application number: **07791066.9**

(22) Date of filing: **20.07.2007**

(51) Int Cl.:
***C07H 19/073*** *(2006.01)* ***A61K 31/7068*** *(2006.01)*
***A61P 35/00*** *(2006.01)*

(86) International application number:
**PCT/JP2007/064328**

(87) International publication number:
**WO 2008/010571 (24.01.2008 Gazette 2008/04)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **21.07.2006 JP 2006199565**

(71) Applicants:
- **TAIHO PHARMACEUTICAL COMPANY, LIMITED Tokyo 101-0054 (JP)**
- **Sasaki, Takuma Nagoya-shi, Aichi 466-0812 (JP)**
- **Matsuda, Akira Sapporo-shi, Hokkaido 001-0024 (JP)**

(72) Inventor: **DOHI, Suguru Hanno-shi Saitama 357-8527 (JP)**

(74) Representative: **HOFFMANN EITLE Patent- und Rechtsanwälte Arabellastrasse 4 81925 München (DE)**

(54) **2'-CYANOPYRIMIDINE NUCLEOSIDE COMPOUND**

(57) The present invention provides a pyrimidine nucleoside compound represented by the following formula (1):

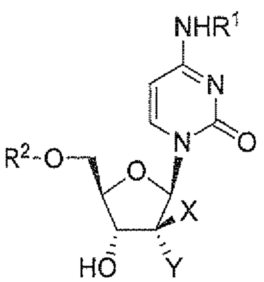

(1)

[wherein one of X and Y is cyano and the other is hydrogen; and i) $R^1$ is hydrogen, and $R^2$ is alkyloxycarbonyl wherein the alkyl moiety is straight or branched $C_{5-12}$ alkyl (the alkyl may be substituted), or alternatively, ii) $R^1$ is alkyloxycarbonyl wherein the alkyl moiety is straight or branched $C_9$ alkyl or alkenyloxycarbonyl wherein the alkenyl moiety is straight or branched $C_9$ alkenyl, and $R^2$ is hydrogen] or a salt thereof. The pyrimidine nucleoside compound or a salt thereof of the present invention shows a higher antitumor effect than existing pyrimidine nucleoside compounds.

[Figure 1]

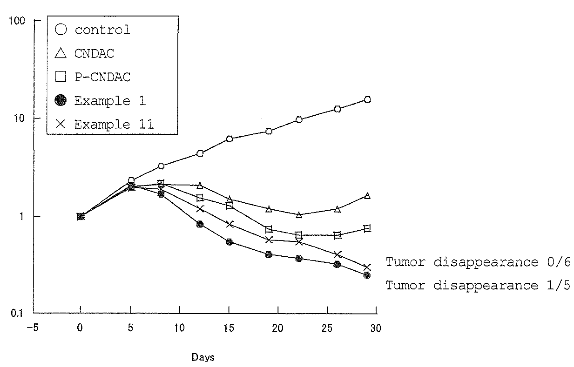


EP 2 045 256 A1

## Description

TECHNICAL FIELD

The present invention relates to a 2'-cyanopyrimidine nucleoside compound or a salt thereof, which has an excellent antitumor effect.

BACKGROUND OF THE INVENTION

Cancers characterized by cell proliferation disorder remain difficult to treat, and there is a demand for the development of drugs with high therapeutic effects. Since nucleic acid biosynthesis is essential to cell proliferation, nucleic acid antimetabolites which inhibit nucleic acid metabolism have been energetically developed.

Under these circumstances, cytidine-based nucleic acid antimetabolites have been actively created. For example, cytarabine (Nonpatent document 1), ancitabine (Nonpatent document 2), cytarabine ocphosphate (Nonpatent document 3), gemcitabine (Patent document 1), and the like have been developed, and are being used for clinical treatment.

These compounds inhibit DNA polymerases or ribonucleotide reductases and thereby inhibit DNA synthesis, thus exhibiting antitumor activity. Although the clinical treatment using these drugs has achieved some positive results, cytarabine, ancitabine, and cytarabine ocphosphate do not have therapeutic effects on solid cancers (Nonpatent document 4). Moreover, the use of gemcitabine is limited to specific types of cancers (Nonpatent document 4). Thus, the antitumor activity of cytidine-based nucleic acid antimetabolites is not satisfactory.

In order to overcome such drawbacks, 2'-cyano-2'-deoxy-1-β-D-arabinofuranosylcytosine (CNDAC) having a DNA strand breaking activity has been developed. This is expected to exhibit antitumor activity different from that of conventional cytidine-based compounds (Patent document 2, Nonpatent documents 5 and 6). Further, 4-N-palmitcyl-2'-cyano-2'-deoxy-1-β-D-arabinofuranosylcytosine (P-CNDAC, Patent document 3, Nonpatent documents 7 and 8) and 5'-phosphatidylpyrimidine nucleotide (Patent document 4) have been developed as oral formulations. These CNDAC compounds have exhibited remarkable antitumor activity (Nonpatent documents 5 to 8). Nonpatent document 5 further indicates that CNDAC compounds, including P-CNDAC, substituted at the 4-N position are useful for the treatment of pancreatic cancer. However, these existing CNDAC compounds have not been placed on the market yet.

One of the problems with such existing oral antitumor agents is their low water solubility. When oral antitumor agents having low water solubility are used, the AUC (area under the curve of the plasma concentration of the drug) or maximum serum concentration is saturated. Administration of such oral antitumor agents to a human causes various problems. For example, no linearity can be seen, the serum concentration does not increase, etc. Therefore, as oral antitumor agents, those having high water solubility and excellent pharmacokinetics are desirable.

Accordingly, the development and market introduction of cytidine-based oral antitumor agents which have excellent water solubility and pharmacokinetics, and exhibit still higher antitumor activity are strongly desired.


[Patent document 1] Japanese Examined Patent Publication No. H06-37394
[Patent document 2] Japanese Patent No. 2559917
[Patent document 3] Japanese Patent No. 2569251
[Patent document 4] Japanese Unexamined Patent Publication No. H07-179491
[Patent document 5] International Patent Publication No. 2005000204
[Nonpatent document 1] Evance, J.S. et al., Proc. Soc. Exp. Bio. Med., 106, 350 (1961)
[Nonpatent document 2] Hoshi, A. et al., Gann, 67, 725 (1972)
[Nonpatent document 3] Kodama, K. et al., Jpn. J. Cancer Res., 80, 679-685 (1989)
[Nonpatent document 4] Matsuda, A., et al., Cancer Sci., 95, 105-111 (2004)
[Nonpatent document 5] Matsuda, A., et al., J. Med. Chem., 34, 2919-2922 (1991)
[Nonpatent document 6] Azuma, A., et al., J. Med. Chem., 36, 4183-4189 (1993)
[Nonpatent document 7] Matsuda, Akira; Sasaki, Takuma: Tanpakushitsu Kakusan Kouso (proteins, nucleic acids, and enzymes), 43, 1981-1989 (1998)
[Nonpatent document 8] Katz, M.H. et al., Cancer Res., 64, 1828-1833 (2004)


DISCLOSURE OF THE INVENTION

PROBLEMS TO BE SOLVED BY THE INVENTION

The object of the present invention is to provide a novel pyrimidine nucleoside compound which has a higher antitumor effect than existing pyrimidine nucleoside compounds.

MEANS FOR SOLVING THE PROBLEMS

The present inventors conducted extensive research to solve the above problems and found that 2'-cyanopyrimidine nucleoside compounds represented by formula (1) given below and salts thereof, orally administered, have high water solubility and excellent bioavailability, and exhibit excellent antitumor activity. The present invention was accomplished based on these findings.

Specifically, the present invention provides a pyrimidine nucleoside compound represented by formula (1):

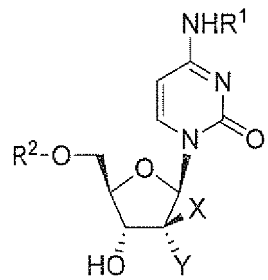
(1)

wherein one of X and Y is cyano and the other is hydrogen;
$R^1$ is hydrogen, alkyloxycarbonyl wherein the alkyl moiety is straight or branched $C_9$ alkyl, or alkenyloxycarbonyl wherein the alkenyl moiety is straight or branched $C_9$ alkenyl;
$R^2$ is hydrogen or alkyloxycarbonyl wherein the alkyl moiety is straight or branched $C_{5-12}$ alkyl (the alkyl may be substituted), provided that:

i) when $R^1$ is hydrogen, $R_2$ is alkyloxycarbonyl wherein the alkyl moiety is straight or branched $C_{5-12}$ alkyl (the alkyl may be substituted), and
ii) when $R^1$ is alkyloxycarbonyl wherein the alkyl moiety is straight or branched $C_9$ alkyl or alkenyloxycarbonyl wherein the alkenyl moiety is straight or branched $C_9$ alkenyl, $R^2$ is hydrogen;

or a salt thereof.

The present invention also provides a pharmaceutical composition containing an effective amount of a compound represented by formula (1) or a salt thereof and a pharmaceutical carrier.

The present invention also provides an antitumor agent containing an effective amount of a compound represented by formula (1) or a salt thereof and a pharmaceutical carrier.

The present invention provides use of a compound represented by formula (1) or a salt thereof for producing a pharmaceutical composition.

The present invention also provides use of a compound represented by formula (1) or a salt thereof for producing an antitumor agent.

The present invention also provides a compound represented by formula (1) or a salt thereof for use in the prevention and/or treatment of a disease.

The present invention also provides a compound represented by formula (1) or a salt thereof for use in the prevention and/or treatment of a tumor.

The present invention also provides a method for preventing and/or treating a disease, the method comprising administering an effective amount of a compound represented by formula (1) or a salt thereof to a patient.

The present invention also provides a method for preventing and/or treating a tumor, the method comprising administering an effective amount of a compound represented by formula (1) or a salt thereof to the patient.

In the present invention, compounds represented by formula (1) and salts thereof may be used alone or in combination.

EFFECT OF THE INVENTION

The 2'-cyanopyrimidine nucleoside compound or a salt thereof of the present invention exhibits excellent antitumor activity. Further, the 2'-cyanopyrimidine nucleoside compound or a salt thereof of the present invention has excellent water solubility, oral absorbability (orally administered absorbability) and bioavailability, and is also useful as an antitumor agent in terms of pharmacokinetics.

BRIEF DESCRIPTION OF THE DRAWINGS

Figure 1 shows a graph indicating the relation between the number of administration days and the relative tumor volume (RTV) with respect to Compounds 1 and 11 of the present invention, CNDAC, and P-CNDAC.

BEST MODE FOR CARRYING OUT THE INVENTION

Novel Pyrimidine Nucleoside Compound or a Salt Thereof

The novel pyrimidine nucleoside compound or a salt thereof of the present invention is represented by the above formula (1). When the 5' position is a hydroxy group, the 4-N position is an alkyloxycarbonyl group or an alkenyloxycarbonyl group, and when the 4-N position is an amino group, the 5' position is an alkyloxycarbonyl group. Although International Patent Application No. PCT/JP06/301483 discloses a compound wherein the 4-N position is an amino group and the 5' position is a silyl group, this is different from the compound of the present invention wherein the 5' position is an alkyloxycarbonyl group as described above.

Patent document 1 describes a 2'-deoxyarabinofuranosylcytosine derivative substituted with fluorine at the 2' position. However, Patent document 1 nowhere describes a CNDAC compound. A CNDAC compound having an alkyloxycarbonyl group or an alkenyloxycarbonyl group at the 4-N position or having an alkyloxycarbonyl group at the 5' position like the compound of the present invention is not disclosed therein.

Patent document 2 describes a CNDAC compound having, at the 4-N position, an alkyloxycarbonyl group wherein the alkyl moiety has one to four carbon atoms or an alkenyloxycarbonyl group wherein the alkenyl moiety has two to four carbon atoms. However, Patent document 2 nowhere describes a CNDAC compound having a $C_9$ alkyloxycarbonyl group or alkenyloxycarbonyl group at the 4-N position, or having an alkyloxycarbonyl group at the 5' position like the compound of the present invention.

Patent document 3 describes a CNDAC compound having acyl groups at the 4-N position and 5' position. However, Patent document 3 nowhere describes a CNDAC compound having an alkyloxycarbonyl group or alkenyloxycarbonyl group at the 4-N position, or having an alkyloxycarbonyl group at the 5' position like the compound of the present invention.

Patent document 4 describes 5'-phosphatidyl pyrimidine nucleotide, but nowhere describes a compound having an alkyloxycarbonyl group or alkenyloxycarbonyl group at the 4-N position, or having an alkyloxycarbonyl group at the 5' position like the compound of the present invention.

Patent document 5 describes that CNDAC compounds substituted at the 4-N position are useful for the treatment of pancreatic cancer. However, patent document 5 nowhere specifically describes a CNDAC compound having an alkyloxycarbonyl group or alkenyloxycarbonyl group at the 4-N position, or having an alkyloxycarbonyl group at the 5' position like the compound of the present invention.

Examples of "alkyloxycarbonyl wherein the alkyl moiety is straight or branched $C_9$ alkyl" represented by $R^1$ in formula (1) include n-nonyloxycarbonyl, nonan-2-yloxycarbonyl, nonan-3-yloxycarbonyl, nonan-4-yloxycarbonyl, nonan-5-yloxycarbonyl, 2-methyloetan-1-yloxycarbanyl, 2-methyloctan-2-yloxycarbonyl, 2-methyloctan-3-yloxycarbonyl, 2-methyloctan-4-yloxycarbonyl, 7-methyloctan-4-yloxycarbonyl, 7-methyloctan-3-yloxycarbonyl, 7-methyloctan-2-yloxycarbonyl, 7-methyloctan-l-yloxycarbonyl, 3-methyloctan-1-yloxycarbonyl, 3-methyloctan-2-yloxyoarbonyl, 3-methyloctan-3-yloxycarbonyl, 3-methyloctan-4-yloxycarbonyl, 6-methyloctan-4-yloxycarbonyl, 6-methyloctan-3-yloxycarbonyl, 6-methyloctan-2-yloxycarbonyl, 6-methyloctan-1-yloxycarbonyl, 4-methyloctan-1-yloxycarbonyl, 4-methyloctan-2-yloxycarbonyl, 4-methyloctan-3-yloxycarbonyl, 4-methyloctan-4-yloxycarbonyl, 5-methyloctan-4-yloxycarbonyl, 5-methyloctan-3-yloxyoarbonyl, 5-methyloctan-2-yloxycarbonyl, 5-methyloctan-1-yloxycarbonyl, 2,2-dimethylheptan-1-yloxycarbonyl, 2,2-dimethylheptan-3-yloxycarbonyl, 2,2-dimethylheptan-4-yloxycarbonyl, 6,6-dimethylheptan-3-yloxycarbonyl, 6,6-dimethylheptan-2-yloxycarbonyl, 6,6-dimethxylheptan-1-yloxycarbonyl, 2,3-dimethylheptan-1-yloxycarbonyl, 2,3-dimethylheptan-2-yloxycarbonyl, 2,3-dimethylheptan-3-yloxycarbonyl, 2,3-dimethylheptan-4-yloxycarbonyl, 5,6-dimethylheptan-3-yloxycarbonyl, 5,6-dimethylheptan-2-yloxycarbonyl, 5,6-dimethylheptan-1-yloxycarbonyl, 2,4-dimethylheptan-1-yloxycarbonyl, 2,4-dimethylheptan-2-yloxycarbonyl, 2,4-dimethylheptan-3-yloxycarbonyl, 2,4-dimethylheptan-4-yloxycarbonyl, 4,6-dimethylheptan-3-yloxycarbonyl, 4,6-dimethylheptan-2-yloxycarbonyl, 4,6-dimethylheptan-1-yloxycarbonyl, 2,5-dimethylheptan-1-yloxycarbonyl, 2,5-dimethylheptan-2-yloxycarbonyl, 2,5-dimethylheptan-3-yloxycarbonyl, 2,5-dimethylheptan-4-yloxycarbonyl, 3,6-dimethylheptan-3-yloxycarbonyl, 3,6-dimethylheptan-2-yloxycarbonyl, 3,6-dimethylheptan-1-yloxycarbonyl, 2,6-dimethylheptan-1-yloxycarbonyl, 2,6-dimethylheptan-2-yloxycarbonyl, 2,6-dimethylheptan-3-yloxycarbanyl, 2,6-dimethylheptan-4-yloxycarbonyl, 3,3-dimethylheptan-1-yloxycarbonyl, 3,3-dimethylheptan-2-yloxycarbonyl, 3,3-dimethylheptan-4-yloxycarbonyl, 5,5-dimethylheptan-3-yloxycarbonyl, 5,5-dimethylheptan-2-yloxycarbonyl, 5,5-dimethylheptan-1-yloxycarbonyl, 3,4-dimethylheptan-1-yloxycarbonyl, 3,4-dimethylheptan-2-yloxycarbonyl, 3,4-dimethylheptan-3-yloxycarbonyl, 3,4-dimethylheptan-4-yloxycarbonyl, 4,5-dimethylheptan-3-yloxycarbonyl, 4,5-dimethylheptan-2-yloxycarbonyl, 4,5-dimethylheptan-1-yloxycarbonyl, 3,5-dimethylheptan-1-yloxycarbonyl, 3,5-dimethylheptan-2-yloxycarbonyl, 3,5-dimethylheptan-3-yloxycarbonyl, 3,5-dimethylheptan-4-yloxycarbonyl, 4,4-dimethylheptan-1-yloxycarbonyl, 4,4-dimethylheptan-2-yloxycarbonyl, 4,4-dimethylheptan-3-yloxycarbonyl, 2,2,3-trimethylhexan-1-yloxycarbonyl, 2,2,3-trimethylhexan-3-yloxycarbonyl, 4,5,5-trimethylhexan-3-yloxycarbonyl, 4,5,5-trimethylhexan-2-yloxycarbonyl, 4,5,5-trimethylhexan-1-yloxycarbonyl, 2,2,4-trimethylhexan-1-yloxycarbonyl, 2,2,4-trimethylhexan-3-yloxycarbonyl, 3,5,5-trimethylhexan-3-yloxycarbonyl, 3,5,5-trimethylhexan-2-yloxycarbonyl, 3,5,5-trimethylhexan-1-yloxycarbonyl, 2,2,5-trimethylhexan-1-yloxycarbonyl, 2,2,5-trimethylhexan-3-yloxycarbonyl, 2,5,5-trimethylhexan-3-yloxycarbonyl, 2,5,5-trimethylhexan-2-yloxycarbonyl, 2,5,5-trimethylhexan-1-yloxycarbonyl, 2,3,3-trimethylhexan-1-yloxycarbonyl, 2,3,3-trimethylhexan-2-yloxycarbonyl, 4,4,5-trimethylhexan-3-yloxycarbonyl, 4,4,5-trimethylhexan-2-yloxycarbonyl, 4,4,5-trimethylhexan-1-yloxycarbonyl, 2,3,4-trimethylhexan-1-yloxycarbonyl, 2,3,4-trimethylhexan-2-yloxycarbonyl, 2,3,4-trimethylhexan-3-yloxycarbonyl, 3,4,5-trimethylhexan-3-yloxycarbonyl, 3,4,5-trimethylhexan-2-yloxycarbonyl, 3,4,5-trimethylhexan-1-yloxycarbonyl, 2,3,5-trimethylhexan-1-yloxycarbonyl, 2,3,5-trimethylhexan-2-yloxycarbonyl, 2,3,5-trimethylhexan-3-yloxycarbonyl, 2,4,5-trimethylhexan-3-yloxycarbonyl, 2,4,5-trimethylhexan-2-yloxycarbonyl, 2,4,5-trimethylhexan-1-yloxycarbonyl, 2,4,4-trimethylhexan-1-yloxycarbonyl, 2,4,4-trimethylhexan-2-yloxycarbonyl, 2,4,4-trimethylhexan-3-yloxycarbonyl, 3,3,5-trimethylhexan-2-yloxycarbanyl, 3,3,5-trimethylhexan-1-yloxycarbonyl, 3-ethyl-2-methylhexan-1-yloxycarbonyl, 4-ethyl-2-methylhexan-1-yloxycarbonyl, 2-ethyl-3-methylhexan-1-yloxycarbonyl, 3-ethyl-3-methylhexan-1-yloxycarbanyl, 3-ethyl-3-methylhexan-2-yloxycarbonyl, 4-ethyl-4-methylhexan-3-yloxycarbonyl, 4-ethyl-4-methylhexan-2-yloxycarbonyl, 4-ethyl-4-methylhexan-1-yloxycarbonyl, 4-ethyl-3-methylhexan-1-yloxycarbonyl, 4-ethyl-3-methylhexan-2-yloxycarbonyl, 4-ethyl-3-methylhexan-3-yloxycarbonyl, 3-ethyl-4-methylhexan-3-yloxycarbonyl, 3-ethyl-4-methylhexan-2-yloxycarbonyl, 3-ethyl-4-methylhexan-1-yloxycarbonyl, 2-ethyl-4-methylhexan-1-yloxycarbonyl, 2-ethyl-5-methylhexan-1-yloxycarbonyl, 3-ethyl-5-methylhexan-1-yloxycarbonyl, 2-propylhexan-1-yloxycarbonyl, 3-propylhexan-1-yloxycarbonyl, 4-propylhexan-1-yloxycarbonyl, 2-isopropylhexan-1-yloxycarbonyl, 3-isopropylhexan-1-yloxycarbonyl, 4-isopropylhexan-1-yloxycarbonyl, 2,2,3,3-tetramethylpentan-1-yloxycarbonyl, 3,3,4,4-tetramethylpentan-1-yloxycarbonyl, 3,3,4,4-tetramethylpantan-2-yloxycarbonyl, 2,2,3,4-tetramethylpentan-1-yloxycarbonyl, 2,2,3,4-tetramethylpentan-3-yloxycarbanyl, 2,3,4,4-tetramethylpentan-2-yloxycarbonyl, 2,2,4,4-tetramethylpentan-3-yloxycarbonyl, 2,3,3,4-tetramethylpentan-1-yloxycarbonyl, 2-ethyl-2,3-dimethylpentan-l-yloxycarbonyl, 2-ethyl-2,4-dimethylpentan-1-yloxycarbonyl, 2-ethyl-3,3-dimethylpentan-1-yloxycarbonyl, 2-ethyl-3,4-dimethylpentan-1-yloxycarbonyl, 2-ethyl-4,4-dimethylpentan-1-yloxycarbonyl, 3-ethyl-2,2-dimethylpentan-1-yloxycarbonyl, 3-ethyl-2,3-dimethylpentan-1-yloxycarbonyl, 3-ethyl-2,4-dimethylpentan-1-yloxycarbonyl, 3-ethyl-3,4-dimethylpentan-1-yloxycarbonyl, 3-ethyl-4,4-dimethylpentan-1-yloxycarbonyl, 3-ethyl-3,4-dimethylpentan-2-yloxycarbonyl, 2,2-diethylpentan-1-yloxycarbonyl, 3,3-diethylpentan-1-yloxycarbonyl, 3,3-diethylpentan-1-yloxycarbonyl, 2-methyl-2-propylpentan-1-yloxycarbonyl, 4-methyl-2-propylpentan-1-yloxycarbonyl, 2-isoprnpyl-2-methylpentan-1-yloxycarbonyl, 2-isopropyl-3-methylpentan-1-yloxycarbonyl, 2-isopropyl-4-methylpentan-1-yloxycarbonyl, etc. n-Nonyloxycarbonyl and nonan-5-yloxycarbonyl are preferable, and n-nonyloxycarbonyl is more preferable.

Examples of "alkenyloxycarbonyl wherein the alkenyl moiety is straight or branched $C_9$ alkenyl" represented by $R^1$ in formula (1) include 8-nonen-l-yloxycarbonyl, (E)-7-nonen-1-yloxycarbonyl, (Z)-7-nonen-1-yloxycarbonyl, (E)-6-nonen-1-yloxycarbonyl, (Z)-6-nonen-1-yloxycarbonyl, (E)-5-nonen-1-yloxycarbonyl, (Z)-5-nonen-1-yloxycarbonyl, (E)-4-nonen-1-yloxycarbonyl, (Z)-4-nonen-1-yloxycarbonyl, (E)-3-nonen-1-yloxycarbonyl, (Z)-3-nonen-1-yloxycarbonyl, (E)-2-nonen-1-yloxycarbonyl, (Z)-2-nonen-1-yloxycarbonyl, (E)-1-nonen-1-yloxycarbonyl, (Z)-1-nonen-1-yloxycarbonyl, 1-nonen-3-yloxycarbonyl, 1-nonen-4-yloxycarbonyl, 1-nonen-5-yloxycarbonyl, 8-nonen-4-yloxycarbonyl, 8-nonen-3-yloxycarbonyl, 8-nonen-2-yloxycarbonyl, (E)-7-nonen-2-yloxycarbonyl, (Z)-7-nonen-2-yloxycarbonyl, (E)-7-nonen-3-yloxycarbonyl, (Z)-7-nonen-5-yloxycarbonyl, (E)-2-nonen-5-yloxycarbonyl, (Z)-2-nonen-5-yloxycarbonyl, (E)-2-nonen-4-yloxycarbonyl, (Z)-2-nonen-4-yloxycarbonyl, etc. Alkenyloxycarbonyl wherein the alkenyl moiety is straight $C_9$ alkenyl is preferable, and alkenyloxycarbonyl wherein the alkenyl moiety is straight $C_9$ alkenyl having a stereochemistry of Z is more preferable. (Z)-6-Nonen-1-yloxycarbonyl, (Z)-3-nonen-1-yloxycarbonyl, and (Z)-2-nonen-1 yloxycarbonyl, are still more preferable.

Examples of substituents in "alkyloxycarbonyl wherein the alkyl moiety is straight or branched $C_{5-12}$ alkyl (the alkyl may be substituted)" represented by $R^2$ in formula (1) include hydroxy, methoxy, ethoxy, isopropoxy, and like $C_{1-3}$ alkoxy; amino; chlorine, bromine, and like halogen atoms; cyano; nitro; etc. The number of substituents is preferably 1.

Examples of "alkyloxycarbonyl" in "alkyloxycarbonyl wherein the alkyl moiety is straight or branched $C_{5-12}$ alkyl (the alkyl, may be substituted)" represented by $R^2$ in formula (1) include n-pentylaxycarbonyl, n-hexyloxycarbonyl, n-heptyloxycarbonyl, n-octyloxycarbonyl, n-nonyloxycarbonyl, n-decyloxycarbonyl, n-undecyloxycarbonyl, n-dodecyloxycarbonyl, pentan-3-yloxycarbonyl, heptan-4-yloxycarbonyl, nonan-5-yloxycarbonyl, isopentyloxycarbonyl, neopentyloxycarbonyl, 4-methylpentyloxycarbonyl, 3-methylpentyloxycarbonyl, 2-methylpentyloxycarbonyl, 1-methylpentyloxycarbonyl, 3,3-dimethylbutyloxycarbonyl, 2,2-dimethylbutyloxycarbonyl, 1,1-dimethylbutyloxycarbonyl, 1,2-dimethylbutyloxycarbonyl, 1,3-dimethylbutyloxycarbonyl, 2,3-dimethylbutyloxycarbonyl, 2-ethylbutyloxycarbonyl, 1-methylhexyloxycarbonyl, 2-methylhexyloxycarbonyl, 3-methylhexyloxycarbonyl, 4-methylhexyloxycarbonyl, 5-methylhexyloxycarbonyl,

4,4-dimethylpentyloxycarbonyl, 1-methylheptyloxycarbonyl, 2-methylheptyloxycarbonyl, 3-methylheptyloxycarbonyl, 4-methylheptyloxycarbonyl, 5-methylheptyloxycarbonyl, 6-methylheptyloxycarbonyl, 1-propylpentyloxycarbonyl, 2-ethylhexyloxycarbonyl, 5,5-dimethylhexyloxycarbonyl, 3-methyloctyloxycarbonyl, 4-methylactylaxycarbonyl, 5-methyloctyloxycarbonyl, 6-methyloctyloxycarbonyl, 1-propylhexyloxycarbonyl, 2-ethylheptyloxycarbonyl, 6,6-dimethylheptyloxycarbonyl, 1-methylnonyloxycarbonyl, 3-methylnonyloxycarbonyl, 8-methylnonyloxycarbonyl, 3-ethyloctyloxycarbonyl, 3,7-dimethyloctyloxycarbonyl, 7,7-dimethyloctyloxycarbonyl, 4,8-dimethylnonyloxycarbonyl, tert-octyl, etc. Alkyloxycarbonyl wherein the alkyl moiety is straight or branched non-substituted $C_{5-12}$ alkyl, is preferable, alkyloxycarbonyl wherein the alkyl moiety is straight or branched non-substituted $C_{6-9}$ alkyl is more preferable, and alkyloxycarbonyl wherein the alkyl moiety is straight non-substituted $C_{6-8}$ alkyl and alkyloxycarbonyl wherein the alkyl moiety is branched non-substituted $C_9$ alkyl are still more preferable. n-Hexyloxycarbonyl, n-heptyloxycarbonyl, n-octyloxycarbonyl, and nonan-5-yloxycarbonyl are especially preferable.

<Preferable Pyrimidine Nucleoside Compound>

Compounds (A) to (G) listed below are preferable in the present invention.

In the present invention, the following Compound (A) is preferable, Compound (B) is more preferable, and Compound (C) is especially preferable:

(A) a compound represented by formula (1) wherein one of X and Y is cyano and the other is hydrogen; $R^1$ is hydrogen; and $R^2$ is alkyloxycarbonyl wherein the alkyl moiety is straight or branched non-substituted $C_{5-12}$ alkyl;
(B) a compound represented by formula (1) wherein one of X and Y is cyano and the other is hydrogen; $R^1$ is hydrogen; and $R^2$ is alkyloxycarbonyl wherein the alkyl moiety is straight non-substituted $C_{6-8}$ alkyl, or alkyloxycarbonyl wherein the alkyl moiety is branched non-substituted $C_9$ alkyl; and
(C) a compound represented by formula (1) wherein one of X and Y is cyano and the other is hydrogen; $R^1$ is hydrogen; and $R^2$ is n-hexyloxycarbonyl, n-heptyloxycarbonyl, n-octyloxycarbonyl, or nonan-5-yloxycarbonyl.

According to another embodiment of the present invention, the following Compound (D) is preferable, Compound (E) is more preferable, and Compound (F) is especially preferable:

(D) a compound represented by formula (1) wherein one of X and Y is cyano and the other is hydrogen; $R^2$ is hydrogen; and $R^1$ is alkyloxycarbonyl wherein the alkyl moiety is straight or branched $C_9$ alkyl, or alkenyloxycarbonyl wherein the alkenyl moiety is straight $C_9$ alkenyl;
(E) a compound represented by formula (1) wherein one of X and Y is cyano and the other is hydrogen; $R^2$ is hydrogen; and $R^1$ is n-nonyloxycarbonyl, nonan-5-yloxycarbonyl, or alkenyloxycarbonyl wherein the alkenyl moiety is straight $C_9$ alkenyl having a stereochemistry of Z; and
(F) a compound represented by formula (1) wherein one of X and Y is cyano and the other is hydrogen; $R^2$ is hydrogen; and $R^1$ is n-nonyloxycarbonyl, (Z)-3-nonen-1-yloxycarbonyl, (Z)-6-nonen-1 yloxycarbonyl, or (Z)-2-nonen-1-yloxycarbonyl.

Specific examples of such compounds include:

(G) pyrimidine nucleoside compounds listed in the following (a) to (n):

(a) 4-N-(n-nonyloxycarbonyl)-2'-cyano-2'-deoxy-1-β-D-arabinofuranosylcytosine,
(b) 4-N-(nonan--5-yloxyoarbonyl)-2'-cyano-2'-deoxy-1-β-D-arabinofuranosylcytosine,
(c) 4-N-[(Z)-6-nonen-1-yloxycarbonyl]-2'-cyano-2'-deoxy-1-β-D-arabinofuranosylcytosine,
(d) 4-N-[(Z)-3-nonen-1-yloxycarbonyl]-2'-cyano-2'-deoxy-1-β-D-arabinofuranosylcytosine,
(e) 4-N-[(E)-2-nonen-1-yloxycarbonyl]-2'-cyano-2'-deoxy-1-β-D-arabinofuranosylcytosine,
(f) 4-N-(8-nonen-1-yloxycarbonyl)-2'-cyano-2'-deoxy-1-β-D-arabinofuranosylcytosine,
(g) 4-N-[(Z)-2-nonen-1-yloxycarbonyl]-2'-cyano-2'-deoxy-1-β-D-arabinofuranosylcytosine,
(h) 5'-O-(n-hexylaxyoarbanyl)-2'-cyano-2'-deaxy-1-β-D-arabinofuranosylcytosine,
(i) 5'-O-(n-heptyloxycarbonyl)-2'-cyano-2'-deoxy-1-β-D-arabinofuranosylcytosine,
(j) 5'-O-(n-octyloxycarbonyl)-2'-cyano-2'-deoxy-1-β-D-arabinofuranosylcytosine,
(k) 5'-O-(nonan-5-yloxycarbonyl)-2'-cyano-2'-deoxy-1-β-D-arabinofuranosylcytosine,
(l) 5'-O-(n-nonyloxycarbonyl)-2'-cyano-2'-deoxy-1-β-D-arabinofuranosylcytosine,
(m) 5'-O-(heptan-4-yloxycarbonyl)-2'-cyano-2'-deoxy-1-β-D-arabinofuranosylcytosine, and
(n) 5'-O-(2,6-dimethylheptan-4-yloxycarbonyl)-2'-cyano-2'-deoxy-1-β-D-arabinofuranosylcytosine.

Salts of the pyrimidine nucleoside compound of the present invention may be any pharmaceutically acceptable salts. The compound of the present invention can form, for example, mineral acid salts such as hydrochloride, hydrobromate, sulfate, nitrate, phosphate, etc.; and organic acid salts such as acetate, propionate, tartrate, fumarate, maleate, malate, citrate, methanesulfonate, p-toluenesulfonate, trifluoroacetate, etc. Depending on the kind of substituent, the pyrimidine nucleoside compound of the present invention may be an optical isomer or a geometrical isomer, which are both encompassed by the scope of the present invention. Such an isomer may be separated and used as a single compound, or alternatively, a mixture may also be used as it is. The pyrimidine nucleoside compound of the present invention may also be a solvate typified by a hydrate; an amorphous polymorph; and a crystal polymorph.

The pyrimidine nucleoside compound or a salt thereof of the present invention can be produced by various methods. For example, it can be produced according to Reaction Schemes 1 to 3 described below.

In Reaction Schemes 1 to 3, X and Y are as defined above. $A^1$ and $A^2$ are hydroxy-protecting groups. The hydroxy-protecting groups are not limited insofar as they can be removed under acidic or neutral conditions, and may be any of those usually used as nucleoside-protecting groups. Preferable examples thereof include trisubstituted silyl such as trimethylsilyl, triethylsilyl, tert-butyldimethylsilyl, triisopropylsilyl, dimethylthexylsilyl, tert-butyldiphenylsilyl, etc.; and substituted or non-substituted triarylmethyl such as triphenylmethyl, 4-methoxytriphenylmethyl, 4,4'-dimethoxyphenylmethyl, etc. $A^3$ is an amino-protecting group. The amino-protecting group is not limited insofar as it can be removed under acidic or neutral conditions, and may be any of those usually used as nucleoside-protecting groups. Preferable examples thereof include tert-butyloxycarbonyl, benzyloxycarbonyl, trichloroethoxycarbonyl, acetyl, etc. B represents $-(R^3)(R^4)Si-O-Si(R^5)(R^6)-$. $R^3$, $R^4$, $R^5$, and $R^6$ may be the same or different, and each represent straight or branched $C_{1-4}$ alkyl, preferably isopropyl.

Reaction Scheme 1

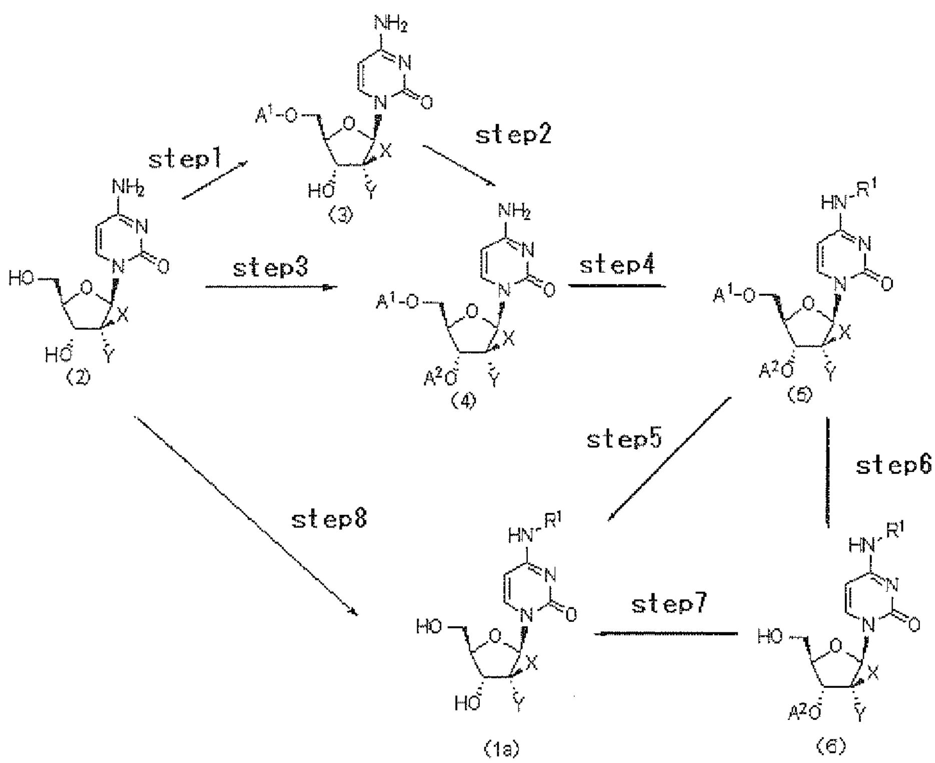


[Production Method 1 (Reaction Scheme 1)]

(Step 1)

In this step, a known pyrimidine nucleoside compound represented by formula (2) or a salt thereof is reacted with a hydroxy-protecting reagent, thereby selectively protecting only the 5' position of the compound represented by formula (2), to produce a compound represented by formula (3) (e.g., Patent documents 3 and 5). Protecting reagents

for this reaction are not limited insofar as they can selectively protect only the 5' position and can be removed under acidic or neutral conditions. Examples thereof include trisubstituted halogenated silanes and triarylmethyl halides represented by $A^1$-$Z^1$ ($A^1$ is as defined above, and $Z^1$ is a halogen atom).

Examples of trisubstituted halogenated silanes include trimethylchlorosilane, triethylchlorosilane, tert-butyldimethylchlorosilane, triisopropylchlorosilane, dimethylthexylchlorosilane, etc.

Examples of triarylmethyl halides include trityl chloride, monomethoxytrityl chloride, dimethoxytrityl chloride, etc.

Solvents for this reaction are not limited insofar as they do not affect the reaction. Examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium carbonate, potassium carbonate, etc. It is possible to use only a base as a solvent. In this reaction, per 1 mol of compound represented by formula (2), the above A'-Z' is used in an amount of about 0.5 to about 20 mol, and preferably about 1 to about 10 mol, and a base is used in an amount of about 0.5 to about 100 mol, and preferably about 1 to about 20 mol. The reaction temperature is -30 to 100 ˚C, and preferably -10 to 60 ˚C. The reaction time is 0.1 to 100 hours, and preferably 1 to 24 hours. The compound represented by formula (3) produced by this reaction may be purified and isolated as required, and may also be used in the subsequent step without purification.

(Step 2)

In this step, a pyrimidine nucleoside compound represented by formula (3) or a salt thereof is reacted with a hydroxy-protecting reagent to produce a compound represented by formula (4) (e.g., Patent documents 3 and 5). Protecting reagents for this reaction are not limited insofar as they can be removed under acidic or neutral conditions. Examples thereof include trisubstituted halogenated silanes and triarylmethyl halides represented by $A^2$-$Z^1$ ($A^2$ and $Z^1$ are as defined above).

Examples of trisubstituted halogenated silanes include trimethylchlorosilane, triethylchlorosilane, tert-butyldimethylchlorosilane, triisopropylchlorosilane, dimethylthexylchlorosilane, etc.

Examples of triarylmethyl halides include trityl chloride, monomethoxytrityl chloride, dimethoxytrityl chloride, etc.

Solvents for this reaction are not limited insofar as they do not affect the reaction. Examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium carbonate, potassium carbonate, etc. It is possible to use only a base as a solvent. In this reaction, per 1 mol of compound represented by formula (3), the above $A^2$-$Z^1$ is used in an amount of about 0.5 to about 20 mol, and preferably about 1 to about 10 mol, and a base is used in an amount of about 0.5 to about 100 mol, and preferably about 1 to about 20 mol. The reaction temperature is -30 to 100 ˚C, and preferably -10 to 60 ˚C. The reaction time is 0.1 to 100 hours, and preferably 2 to 48 hours. The compound represented by formula (4) produced by this reaction may be purified and isolated as required, and may also be used in the subsequent step without purification.

(Step 3)

In this step, a pyrimidine nucleoside compound represented by formula (2) or a salt thereof is reacted with a hydroxy-protecting reagent to produce a compound represented by formula (4) (in this reaction, $A^1$ and $A^2$ are the same substituents) (e.g., Patent documents 3 and 5). Protecting reagents for this reaction are not limited insofar as they can be removed under acidic or neutral conditions. Examples thereof include trisubstituted halogenated silanes and triarylmethyl halides represented by $A^1$-$Z^1$ ($A^1$ and $Z^1$ are as defined above).

Examples of trisubstituted halogenated silanes include trimethylchlorosilane, triethylchlorosilane, tert-butyldimethylchlorosilane, triisopropylchlorosilane, dimethylthexylchlorosilane, etc.

Examples of triarylmethyl halides include trityl chloride, monomethoxytrityl chloride, dimethoxytrityl chloride, etc.

Solvents for this reaction are not limited insofar as they do not affect the reaction. Examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium

carbonate, potassium carbonate, etc. It is possible to use only a base as a solvent. In this reaction, per 1 mol of compound represented by formula (2), the above $A^1$-$Z^1$ is used in an amount of about 1 to about 40 mol, and preferably about 2 to about 20 mol, and a base is used in an amount of about 1 to about 100 mol, and preferably about 4 to about 60 mol. The reaction temperature is -30 to 100 ˚C, and preferably -10 to 60 ˚C. The reaction time is 0.1 to 100 hours, and preferably 1 to 48 hours. The compound represented by formula (4) produced by this reaction may be purified and isolated as required, and may also be used in the subsequent step without purification.

(Step 4)

In this step, a pyrimidine nucleoside compound represented by formula (4) or a salt thereof is reacted with an alkyloxycarbonylating agent or alkenyloxycarbonylating agent represented by $R^{1a}$OCO-$Z^2$ ($Z^2$ represents halogen, p-nitrophenoxy, or 1-H-imidazole-1-yl, and $R^{1a}$ represents straight or branched $C_9$ alkyl or straight or branched $C_9$ alkenyl) or $R^{1a}OCO_2R^{1b}$ ($R^{1b}$ is as defined with respect to $R^{1a}$, or instead represents straight or branched $C_{1-5}$ alkyl, e.g., isobutyl) to produce a compound represented by formula (5).

Any of a wide variety of known alkyloxycarbonylating agents and alkenyloxycarbonylating agents represented by $R^{1a}$OCO-$Z^2$ ($R^{1a}$ and $Z^2$ are as defined above) may be used herein. Examples thereof include alkyl haloformates, alkenyl haloformates, alkyl(4-nitrophenyl)carbonates, alkenyl(4-nitrophenyl)carbonates, 1-H-imidazole-1-carboxylic acid alkyl esters, 1-H-imidazole-1-carboxylic acid alkenyl esters, etc.

Any of a wide variety of known alkyloxycarbonylating agents and alkenyloxycarbonylating agents represented by $R^{1a}OCO_2R^{1b}$ ($R^{1a}$ and $R^{1b}$ are as defined above) may be used herein. Examples thereof include dialkyl dicarbonates, dialkenyl dicarbonates, etc.

Among alkyloxycarbonylating agents and alkenyloxycarbonylating agents represented by $R^{1a}$OCO-$Z^2$ ($P^{1a}$ and $Z^2$ are as defined above) usable in this reaction, alkyl haloformates and alkenyl haloformates may be prepared by known methods. For example, they can be obtained by reacting triphosgene with a corresponding alkyl alcohol or alkenyl alcohol.

Among alkyloxycarbonylating agents and alkenyloxycarbonylating agents represented by $R^{1a}$OCO-$Z^2$ ($R^{1a}$ and $Z^2$ are as defined above) usable in this reaction, alkyl(4-nitrophenyl)carbonates and alkenyl(4-nitrophenyl)carbonates may also be prepared by a known method. For example, they can be obtained by reacting 4-nitrophenyl chloroformate with a corresponding alkyl alcohol or alkenyl alcohol.

Among alkyloxycarbonylating agents and alkenyloxycarbonylating agents represented by $R^{1a}$OCO-$Z^2$ ($R^{1a}$ and $Z^2$ are as defined above) usable in this reaction, 1-H-imidazole-1-carboxylic acid alkyl esters and 1-H-imidazole-1-carboxylic acid alkenyl esters may also be prepared by a known method. For example, they can be obtained by reacting 1,1'-carbonyldiimidazole with a corresponding alkyl alcohol or alkenyl alcohol.

Alkyl haloformates, alkenyl haloformates, alkyl(4-nitrophenyl)carbonates, alkenyl(4-nitrophenyl)carbonates, 1-H-imidazole-1-carboxylic acid alkyl esters, and 1-H-imidazole-1 carboxylic acid alkenyl esters represented by $R^{1a}$OCO-$Z^2$ ($R^{1a}$ and $Z^2$ are as defined above) may be purified and isolated as required, and may also be used in this step without purification.

The reaction may be carried out by a known method. Solvents therefor are not limited insofar as they do not affect the reaction, and examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium carbonate, potassium carbonate, etc. It is possible to use only a base as a solvent. In this reaction, per 1 mol of compound represented by formula (4), the above $R^{1a}$OCO-$Z^2$ ($R^{1a}$ and $Z^2$ are as defined above) is used in an amount of about 0.5 to about 20 mol, and preferably about 1 to about 10 mol, and a base is used in an amount of about 0.5 to about 100 mol, and preferably about 1 to about 20 mol. The reaction temperature is -30 to 100 ˚C, and preferably -10 to 30 ˚C. The reaction time is 0.1 to 100 hours, and preferably 1 to 72 hours. The compound represented by formula (5) produced by this reaction may be purified and isolated as required, and may also be used in the subsequent step without purification.

(Step 5)

In this step, a pyrimidine nucleoside compound represented by formula (5) or a salt thereof is reacted with a deprotecting reagent to produce a compound represented by formula (1a). Solvents used are not limited insofar as they do not affect the reaction, and examples thereof include dichloromethane, chloroform, ethyl acetate, tetrahydrofuran, dioxane, diethyl ether, benzene, toluene, acetone, N,N-dimethylformamide, N,N-dimethylacetamide, dimethyl sulfoxide, water, etc. These solvents may be used alone or in combination. Examples of usable deprotecting reagents include

mineral acids such as hydrochloric acid, hydrobromic acid, sulfuric acid, nitric acid, phosphoric acid, etc.; and organic acids such as trifluoroacetic acid, acetic acid, propionic acid, formic acid, methanesulfonic acid, p-toluenesulfonic acid, etc. These acids may be used in mixture with water. When $A^1$ and $A^2$ in formula (5) are both trisubstituted silyl, any of a wide variety of deprotecting reagents usually used to deprotect silyl groups may be employed. Examples thereof include fluoride ion reagents such as tetrabutylammonium fluoride, hydrogen fluoride, potassium fluoride, etc. In this reaction, per 1 mol of compound represented by formula (5), the deprotecting reagent is used in an amount of about 0.5 to about 200 mol, and preferably about 2 to about 100 mol. The reaction temperature is -30 to 150 ˚C, and preferably -10 to 50 ˚C. The reaction time is 0.1 to 100 hours, and preferably 1 to 48 hours. Steps 3 to 5 may be performed in succession.

(Step 6)

In this step, a pyrimidine nucleoside compound represented by formula (5) or a salt thereof is reacted with a deprotecting reagent, thereby selectively deprotecting only the 5' position of a compound represented by formula (5), to produce a compound represented by formula (6). Solvents used are not limited insofar as they do not affect the reaction, and examples thereof include dichloromethane, chloroform, ethyl acetate, tetrahydrofuran, dioxane, diethyl ether, benzene, toluene, acetone, N,N-dimethylformamide, N,N-dimethylacetamide, dimethyl sulfoxide, water, etc. These solvents may be used alone or in combination. When $A^1$ in formula (5) is trisubstituted silyl, any of a wide variety of deprotecting reagents capable of removing only $A^1$ may be used. Examples thereof include fluoride ion reagents such as tetrabutylammonium fluoride, hydrogen fluoride, potassium fluoride, etc; mineral acids such as hydrochloric acid, hydrobromic acid, sulfuric acid, nitric acid, phosphoric acid, etc; and organic acids such as trifluoroacetic acid, acetic acid, propionic acid, formic acid, methanesulfonic acid, p-toluenesulfonic acid, etc. When $A^1$ in formula (5) is triarylmethyl, any of a wide variety of deprotecting reagents capable of removing only $A^1$ may be used. Examples thereof include mineral acids and organic acids as described above. These acids may be used in mixture with water. In this reaction, per 1 mol of compound represented by formula (5), the above deprotecting reagent is used in an amount of about 0.5 to about 200 mol, and preferably about 1 to about 100 mol. The reaction temperature is -30 to 150 ˚C, and preferably -10 to 50 ˚C. The reaction time is 0.1 to 100 hours, and preferably 0.5 to 24 hours.

(Step 7)

In this step, a pyrimidine nucleoside compound represented by formula (6) or a salt thereof is reacted with a deprotecting reagent to produce a compound represented by formula (1a). Solvents used are not limited insofar as they do not affect the reaction, and examples thereof include dichloromethane, chloroform, ethyl acetate, tetrahydrofuran, dioxane, diethyl ether, benzene, toluene, acetone, N,N-dimethylformamide, N,N-dimethylacetamide, dimethyl sulfoxide, water, etc. These solvents may be used alone or in combination. When $A^2$ in formula (6) is trisubstituted silyl, any of a wide variety of deprotecting reagents usually used to deprotect silyl groups may be employed. Examples thereof include fluoride ion reagents such as tetrabutylammonium fluoride, hydrogen fluoride, potassium fluoride, etc; mineral acids such as hydrochloric acid, hydrobromic acid, sulfuric acid, nitric acid, phosphoric acid, etc; and organic acids such as trifluoroacetic acid, acetic acid, propionic acid, formic acid, methanesulfonic acid, p-toluenesulfonic acid, etc. When $A^2$ in formula (6) is triarylmethyl, any of a wide variety of deprotecting reagents usually used to deprotect triarylmethyl groups may be employed. Examples thereof include mineral acids and organic acids as described above. These acids may be used in mixture with water. In this reaction, per 1 mol of compound represented by formula (6), the above deprotecting reagent is used in an amount of about 0.5 to about 200 mol, and preferably about 1 to about 100 mol. The reaction temperature is -30 to 150 ˚C, and preferably -10 to 50 ˚C. The reaction time is 0.1 to 100 hours, and preferably 1 to 48 hours.

(Step 8)

In this step, an unprotected pyrimidine nucleoside compound represented by formula (2) or a salt thereof is reacted with an alkyloxycarbonylating agent or alkenyloxycarbonylating agent represented by $R^{1a}OCO\text{-}Z^2$ ($R^{1a}$ and $Z^2$ are as defined above) to produce a compound represented by formula (1a), and may be performed in the same manner as in Step 4.

Any of a wide variety of known alkyloxycarbonylating agents and alkenyloxycarbonylating agents represented by $R^{1a}OCO\text{-}Z^2$ ($R^{1a}$ and $Z^2$ are as defined above) may be used herein. Examples thereof include alkyl haloformates, alkenyl haloformates, alkyl(4-nitrophenyl)carbonates, alkenyl(4-nitrophenyl)carbonates, 1-H-imidazole-1-carboxyli-c acid alkyl, esters, 1-H-imidazole-1-carboxylic acid alkenyl esters, etc.

Reaction Scheme 2

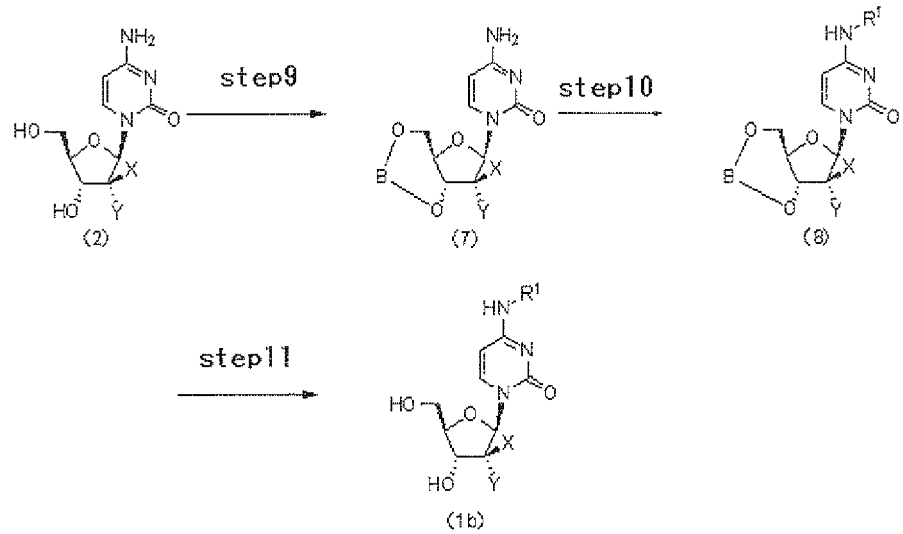

(2) (7) (8) (1b)

[Production Method 2 (Reaction Scheme 2)]

(Step 9)

In this step, a pyrimidine nucleoside compound represented by formula (2) or a salt thereof is reacted with a compound represented by $Z^1$-($R^3$) ($R^4$) Si-O-Si ($R^5$) ($R^6$) -$Z^1$ ($R^3$, $R^4$, $R^5$, $R^6$, and $Z^1$ are as defined above, and $Z^1$ is preferably chlorine or bromine) to produce a compound represented by formula (7). A compound represented by formula (7) can be produced according to a known method (e.g., Patent document 3). Solvents for this reaction are not limited insofar as they do not affect the reaction. Examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium carbonate, potassium carbonate, etc. It is possible to use only a base as a solvent. In this reaction, per 1 mol of compound represented by formula (2), the above compound represented by $Z^1$-($R^3$) ($R^4$) Si-O-Si ($R^5$) ($R^6$)-$Z^1$ is used in an amount of about 0.5 to about 20 mol, and preferably about 1 to about 10 mol, and a base is used in an amount of about 0.5 to about 100 mol, and preferably about 1 to about 20 mol. The reaction temperature is -30 to 100 °C, and preferably 0 to 60 °C. The reaction time is 0.1 to 100 hours, and preferably 1 to 24 hours. The compound represented by formula (7) produced by this reaction may be purified and isolated as required, and may also be used in the subsequent step without purification.

(Step 10)

In this step, a pyrimidine nucleoside compound represented by formula (7) or a salt thereof is reacted with an alkyloxycarbonylating agent or alkenyloxycarbonylating agent represented by $R^{1a}OCO$-$Z^2$ ($R^{1a}$ and $Z^2$ are as defined above) to produce a compound represented by formula (8), and may be performed in the same manner as in Step 4.

Any of a wide variety of known alkyloxycarbonylating agents and alkenyloxycarbonylating agents represented by $R^{1a}OCO$-$Z^2$ ($R^{1a}$ and $Z^2$ are as defined above) may be used herein. Examples thereof include alkyl haloformates, alkenyl haloformates, alkyl (4-nitrophenyl) carbonates, alkenyl (4-nitrophenyl) carbonates, 1-H-imidazole-1-carboxylic acid alkyl esters, 1-H-imidazole-1-carboxylic acid alkenyl esters, etc.

(Step 11)

In this step, a pyrimidine nucleoside compound represented by formula (8) or a salt thereof is reacted with a

hydroxy-deprotecting reagent to produce a compound represented by formula (1b). Solvents used are not limited insofar as they do not affect the reaction, and examples thereof include dichloromethane, chloroform, ethyl acetate, tetrahydrofuran, dioxane, diethylether, benzene, toluene, acetone, N,N-dimethylformamide, N,N-dimethylacetamide, dimethyl sulfoxide, etc. These solvents may be used alone or in combination. Any of a wide variety of deprotecting reagents usually used to deprotect silyl groups may be employed herein. Examples thereof include fluoride ion reagents such as tetrabutylammonium fluoride, hydrogen fluoride, potassium fluoride, etc; mineral acids such as hydrochloric acid, hydrobromic acid, sulfuric acid, nitric acid, phosphoric acid, etc; and organic acids such as trifluoroacetic acid, acetic acid, propionic acid, formic acid, methanesulfonic acid, p-toluenesulfonic acid, etc. These acids may be used in mixture with water. In this reaction, per 1 mol of compound represented by formula (8), the above deprotecting reagent is used in an amount of about 1 to about 200 mol, and preferably about 2 to about 100 mol. The reaction temperature is -30 to 150 ˚C, and preferably -10 to 50 ˚C. The reaction time is 0.1 to 100 hours, and preferably 1 to 48 hours.

Reaction Scheme 3

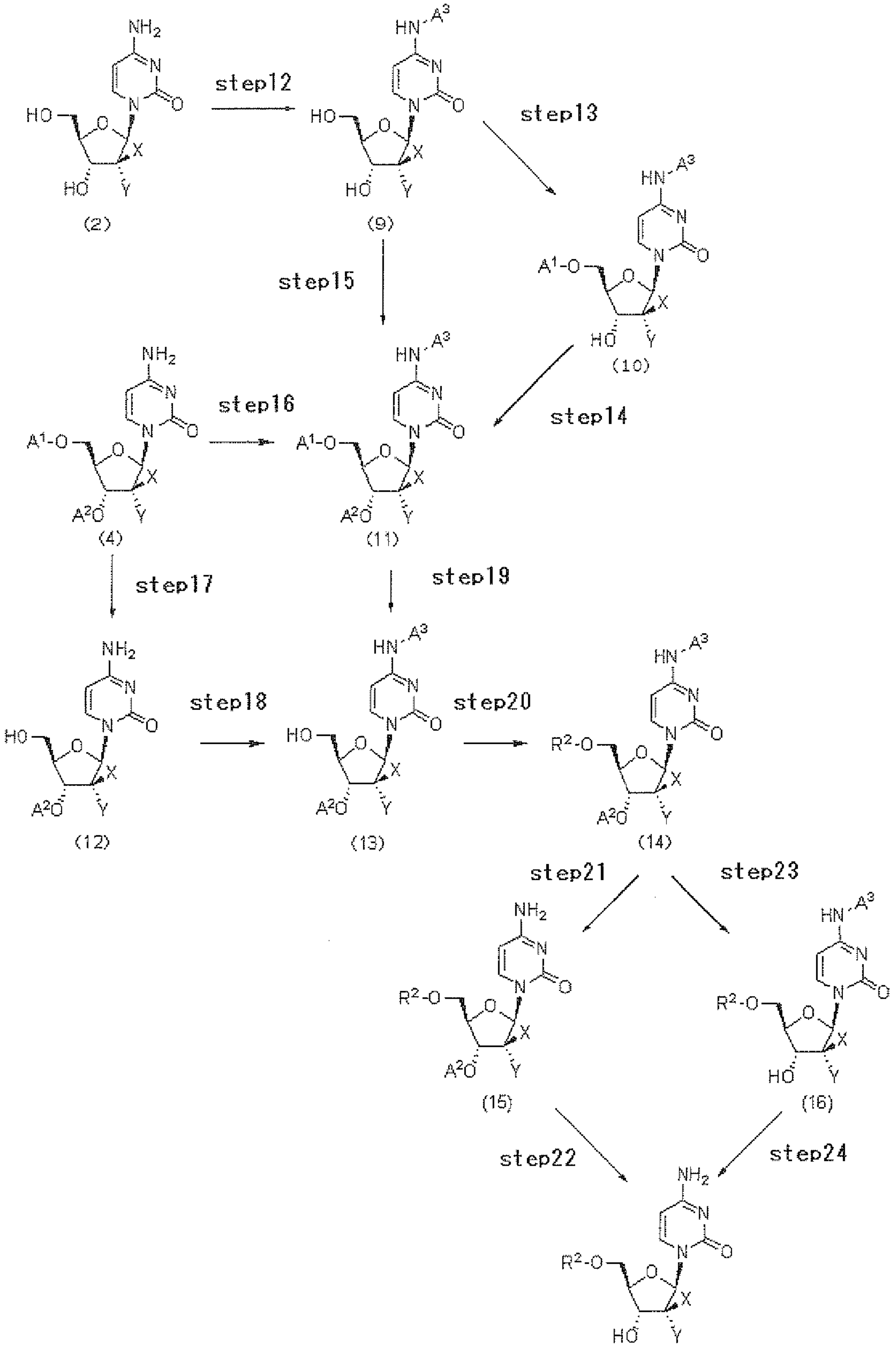

[Production Method 3 (Reaction Scheme 3)]

(Step 12)

In this step, a pyrimidine nucleoside compound represented by formula (2) or a salt thereof is reacted with an amino-protecting reagent to produce a compound represented by formula (9). Solvents used are not limited insofar as they do not affect the reaction, and examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium carbonate, potassium carbonate, etc. It is possible to use only a base as a solvent. Amino-protecting reagents used are not limited insofar as the amino-protecting group can be removed under acidic or neutral conditions. Examples thereof include compounds represented by $A^3$-$Z^1$ ($A^3$ and $Z^1$ are as defined above) and those represented by $A^3$-O-$A^3$ ($A^3$ is as defined above).

Examples of compounds represented by $R^3$-$Z^1$ ($A^3$ and $Z^1$ are as defined above) include haloalkoxy carbonyl halides such as acetic acid halide and trichloroethoxycarbonyl chloride, and alkenyloxycarbonyl halides such as benzyloxycarbonyl chloride, etc.

Examples of compounds represented by $A^3$-O-$A^3$ ($A^3$ is as defined above) include acetic anhydride, di-tert-butyl dicarbonate, etc.

In this reaction, per 1 mol of compound represented by formula (2), the above $A^3$-$Z^1$ or $A^3$-O-$A^3$ is used in an amount of about 0.5 to about 10 mol, and preferably about 1 to about 5 mol. When a base is used, the amount thereof is about 0.5 to about 20 mol, and preferably about 1 to about 10 mol, per 1 mol of compound represented by formula (2). The reaction temperature is -30 to 150 ˚C, and preferably -10 to 70 ˚C. The reaction time is 0.1 to 100 hours, and preferably 1 to 40 hours. The compound represented by formula (9) produced by this reaction may be purified and isolated as required, and may also be used in the subsequent step without purification.

(Step 13)

In this step, a pyrimidine nucleoside compound represented by formula (9) or a salt thereof is reacted with a hydroxy-protecting reagent, thereby selectively protecting only the 5' position of a compound represented by formula (9), to produce a compound represented by formula (10). This step can be performed in the same manner as in Step 1.

(Step 14)

In this step, a pyrimidine nucleoside compound represented by formula (10) or a salt thereof is reacted with a hydroxy-protecting reagent to produce a compound represented by formula (11). This step can be performed in the same manner as in Step 2.

(Step 15)

In this step, a pyrimidine nucleoside compound represented by formula (9) or a salt thereof is reacted with a hydroxy-protecting reagent to produce a compound represented by formula (11) (in this step, $A^1$ and $A^2$ are the same substituents). This step can be performed in the same manner as in Step 3.

(Step 16)

In this step, a pyrimidine nucleoside compound represented by formula (4) or a salt thereof is reacted with an amino-protecting reagent to produce a compound represented by formula (11). This step can be performed in the same manner as in Step 12.

(Step 17)

In this step, a pyrimidine nucleoside compound represented by formula (4) or a salt thereof is reacted with a deprotecting reagent and selectively deprotects only the 5' position of a compound represented by formula (4) to produce a compound represented by formula (12). This step can be performed in the same manner as in Step 6.

(Step 18)

In this step, a pyrimidine nucleoside compound represented by formula (12) or a salt thereof is reacted with an amino-protecting reagent to produce a compound represented by formula (13). This step can be performed in the same manner as in Step 12.

(Step 19)

In this step, a pyrimidine nucleoside compound represented by formula (11) or a salt thereof is reacted with a deprotecting reagent under neutral conditions and deprotects only the 5' position of a compound represented by formula (11) to produce a compound represented by formula (13) (in this step, $A^1$ is trisubstituted silyl). Solvents used are not limited insofar as they do not affect the reaction, and examples thereof include dichloromethane, chloroform, ethyl acetate, tetrahydrofuran, dioxane, diethylether, benzene, toluene, acetone, N,N-dimethylformamide, N,N-dimethylacetamide, dimethyl sulfoxide, etc. These solvents may be used alone or in combination. Any deprotecting reagents usually used to deprotect silyl groups may be employed herein, and examples thereof include fluoride ion reagents, such as tetrabutylammonium fluoride, hydrogen fluoride, potassium fluoride, etc. In this reaction, per 1 mol of compound represented by formula (11), the above deprotecting reagent is used in an amount of about 1 to about 20 mol, and preferably about 2 to about 10 mol. The reaction temperature is -30 to 150 ˚C, and preferably -10 to 50 ˚C. The reaction time is 0.1 to 100 hours, and preferably 1 to 48 hours.

(Step 20)

In this step, a pyrimidine nucleoside compound represented by formula (13) or a salt thereof is reacted with an alkyloxycarbonylating agent or alkenyloxycarbonylating agent represented by $R^{2a}OCO\text{-}Z^2$ to produce a compound represented by formula (14). ($Z^2$ is as defined above, and $R^{2a}$ represents substituted or non-substituted straight or branched $C_{5-12}$ alkyl. When $R^{2a}$ is substituted, the number of substituents is preferably 1, The kind of such substituents is the same as for those that the above alkyloxycarbonyl represented by $R^2$ may have.)

Any of a wide variety of known alkyloxycarbonylating agents and alkenyloxycarbonylating agents represented by $R^{2a}OCO\text{-}Z^2$ ($Z^2$ and $R^{2a}$ are as defined above) may be used herein. Examples thereof include alkyl haloformates, alkenyl haloformates, alkyl (4-nitrophenyl) carbonates, alkenyl(4-nitrophenyl) carbonates, 1-H-imidazole-1-carboxylic acid alkyl esters, 1-H-imidazole-1-carboxylic acid alkenyl esters, etc.

Among alkyloxycarbonylating agents and alkenyloxycarbonylating agents represented by $R^{2a}OCO\text{-}Z^2$ ($Z^2$ and $R^{2a}$ are as defined above) usable in this reaction, alkyl haloformates and alkenyl haloformates may be prepared by a known method. For example, they can be obtained by reacting triphosgene with a corresponding alkyl alcohol or alkenyl alcohol. Among alkyloxycarbonylating agents and alkenyloxycarbonylating agents represented by $R^{2a}OCO\text{-}Z^2$ ($Z^2$ and $R^{2a}$ are as defined above) usable in this reaction, alkyl(4-nitrophenyl)carbonates and alkenyl(4-nitrophenyl)carbonates can also be prepared by a known method. For example, they can be obtained by reacting 4-nitrophenyl chloroformate with a corresponding alkyl alcohol or alkenyl alcohol. Among alkyloxycarbonylating agents and alkenyloxycarbonylating agents represented by $R^{2a}OCO\text{-}Z^2$ ($Z^2$ and $R^{2a}$ are as defined above) usable in this reaction, 1-H-imidazole-1-carboxylic acid alkyl esters and 1-H-imidazole-1-carboxylic acid alkenyl esters may also be prepared by a known method. For example, they can be obtained by reacting 1,1'-carbonyldiimidazole with a corresponding alkyl alcohol or alkenyl alcohol.

Alkyl haloformates, alkenyl haloformates, alkyl (4-nitrophenyl) carbonates, alkenyl(4-nitrophenyl)carbonates, 1-H-imidazole-1-carboxylic acid alkyl esters, and 1-H-imidazole-1-carboxylic acid alkenyl esters represented by $R^{2a}OCO\text{-}Z^2$ ($Z^2$ and $R^{2a}$ are as defined above) may be purified and isolated as required, and may also be used in this step without purification. The reaction may be carried out by a known method. Solvents used are not limited insofar as they do not affect the reaction, and examples thereof include dichloromethane, chloroform, ethyl acetate, tetrahydrofuran, dioxane, diethylether, benzene, toluene, N,N-dimethylformamide, N,N-dimethylacetamide, dimethyl sulfoxide, etc. These solvents may be used alone or in combination. If necessary, a base may be used in this reaction. Examples of usable bases include organic amines such as imidazole, 1-methylimidazole, trimethylamine, triethylamine, tripropylamine, diisopropylethylamine, N-methylmorpholine, pyridine, 4-(N,N-dimethylamino)pyridine, lutidine, collidine, etc.; and inorganic bases such as sodium hydrogen carbonate, sodium carbonate, potassium carbonate, etc. It is possible to use only a base as a solvent. In this reaction, per 1 mol of compound represented by formula (13), the above $R^{2a}OCO\text{-}Z^2$ ($Z^2$ and $R^{2a}$ are as defined above) is used in an amount of about 0.5 to about 20 mol, and preferably about 1 to about 10 mol. When a base is used, the amount thereof is about 0.5 to about 100 mol, and preferably about 1 to about 20 mol, per 1 mol of compound represented by formula (13). The reaction temperature is -30 to 100 ˚C, and preferably -10 to 50 ˚C. The reaction time is 0.1 to 100 hours, and preferably 1 to 24 hours. The compound represented by formula (14) produced by this reaction may be purified and isolated as required, and may also be used in the subsequent step without purification.

(Step 21)

In this step, a pyrimidine nucleoside compound represented by formula (14) or a salt thereof is reacted with a deprotecting reagent for amino-protecting groups to produce a compound represented by formula (15). Deprotecting reagents used are not limited insofar as they can remove only the substituent represented by $A^3$. Examples thereof include, when $A^3$ is acetyl, tert-butoxycarbonyl, or the like, mineral acids such as hydrochloric acid, hydrobromic acid, sulfuric acid, nitric acid, phosphoric acid, etc.; and organic acids such as trifluoroacetic acid, acetic acid, propionic acid, formic acid, methanesulfonic acid, p-toluenesulfonic acid, etc. These acids may be used in mixture with water. When $A^3$ is alkenyloxycarbonyl such as benzyloxycarbonyl, catalytic reduction can be used. Examples of solvents used include dichloromethane, chloroform, ethyl acetate, tetrahydrofuran, dioxane, diethylether, benzene, toluene, N,N-dimethylformamide, N,N-dimethylacetamide, dimethyl sulfoxide, methanol, ethanol, n-propanol, isopropanol, water, etc. These solvents may be used alone or in combination. In this reaction, per 1 mol of compound represented by formula (14), the above deprotecting reagent is used in an amount of about 0.5 to about 200 mol, and preferably about 1 to about 100 mol. The reaction temperature is -30 to 150 ˚C, and preferably -10 to 50 ˚C. The reaction time is 0.1 to 100 hours, and preferably 1 to 24 hours.

(Step 22)

In this step, a pyrimidine nucleoside compound represented by formula (15) or a salt thereof is reacted with a deprotecting reagent for hydroxy-protecting groups to produce a compound represented by formula (1c). This step can be performed in the same manner as in Step 7.

(Step 23)

In this step, a pyrimidine nucleoside compound represented by formula (14) or a salt thereof is reacted with a deprotecting reagent for hydroxy-protecting groups to produce a compound represented by formula (16). This step can be performed in the same manner as in Step 7.

(Step 24)

In this step, a pyrimidine nucleoside compound represented by formula (16) or a salt thereof is reacted with a deprotecting reagent for amino-protecting groups to produce a compound represented by formula (1c). This step can be performed in the same manner as in Step 21.

The compounds of the present invention and other compounds thus obtained can form salts, especially pharmaceutically acceptable salts, according to known methods.

The compounds of the present invention and salts thereof, as well as other compounds and salts thereof, can be purified and isolated by known separation and purification means, such as concentration, solvent extraction, filtration, recrystallization, various chromatography techniques, etc.

Pharmaceutical Composition

The present invention provides a pharmaceutical composition containing an effective amount of at least one compound of the present invention represented by formula (1) or a pharmaceutically acceptable salt thereof.

When used as a medicine, the compound of the present invention is mixed with a pharmaceutical carrier. Depending on the purpose of prevention or treatment, various dosage forms can be employed. The dosage form may be, for example, an oral formulation, injection, suppository, ointment, patch, etc. An oral formulation is preferable. Such dosage forms can be produced according to common preparation methods known to those skilled in the art.

As the pharmaceutical carrier, various organic or inorganic carrier materials commonly used as pharmaceutical raw materials are usable. These are used as excipients, lubricants, binders, or disintegrants in solid formulations, or as solvents, solubilizing agents, suspending agents, isotonizing agents, buffers, or soothing agents in liquid formulations, etc. If necessary, other pharmaceutical additives such as preservatives, antioxidants, colorants, sweeteners, and the like may also be used.

Oral solid formulations are prepared as follows. An excipient, optionally together with a binder, disintegrant, lubricant, colorant, sweetening/flavoring agent, etc., are added into the compound of the present invention to produce tablets, coated tablets, granules, powders, capsules, or the like using a standard method. As such additives, those commonly used in the field may be employed. Examples of usable excipients include lactose, sucrose, sodium chloride, glucose, starch, calcium. carbonate, kaolin, microcrystalline cellulose, silicic acid, etc. Examples of usable binders include water, ethanol, propanol, simple syrup, liquid glucose, liquid starch, liquid gelatin, carboxymethyl cellulose, hydroxypropyl

cellulose, hydroxypropyl starch, methyl cellulose, ethyl cellulose, shellac, calcium phosphate, polyvinylpyrrolidone, etc. Examples of usable disintegrants include dry starch, sodium alginate, agar powder, sodium hydrogen carbonate, calcium carbonate, sodium lauryl sulfate, stearic acid monoglyceride, lactose, etc. Examples of usable lubricants include purified talc, stearic acid salts, borax, polyethylene glycol, etc. Examples of usable colorants include titanium oxide, iron oxide, etc. Examples of usable sweetening/flavoring agents include sucrose, wild orange peel, citric acid, tartaric acid, etc.

Liquid oral formulations are produced as follows. A sweetening/flavoring agent, buffer, stabilizer, etc., are added into the compound of the present invention to produce internal liquid medicines, syrups, elixirs, or the like using a standard method. In this case, sweetening/flavoring agents as described above are usable. Examples of usable buffers include sodium citrate, etc., and examples of usable stabilizers include tragacanth, gum arabic, gelatin, etc.

Injections are prepared as follows. A pH adjuster, buffer, stabilizer, isotonizing agent, topical anesthetic, etc., are added into the compound of the present invention to produce subcutaneous injections, intramuscular injections, or intravenous injections using a standard method. Examples of usable pH adjusters and buffers in this case include sodium citrate, sodium acetate, sodium phosphate, etc. Examples of usable stabilizers include sodium pyrosulfite, EDTA, thioglycolic acid, thiolactic acid, etc. Examples of usable topical anesthetics include procaine hydrochloride, lidocaine hydrochloride, etc. Examples of usable isotonizing agents include sodium chloride, glucose, etc.

Suppositories are prepared as follows. Pharmaceutical carriers known in the art, such as polyethylene glycol, lanolin, cacao butter, fatty acid triglyceride, etc., are added into the compound of the present invention, optionally together with Tween™ and like surfactants, etc., followed by production using a standard method.

Ointments are prepared as follows. An ordinary base, stabilizer, wetting agent, preservative, etc., are added as required into the compound of the present invention, and mixed and formulated using a standard method. Examples of usable bases include liquid paraffin, white petrolatum, white beeswax, octyldodecyl alcohol, paraffin, etc. Examples of usable preservatives include methyl parahydroxybenzoate, ethyl parahydroxybenzoate, propyl parahydroxybenzoate, etc.

Patches can be prepared by coating an ordinary support with the above ointment, cream, gel, paste, etc., using a standard method. Examples of usable supports include woven or nonwoven fabrics made from cotton, staple fibers, and chemical fibers; and films and foam sheets of soft vinyl chloride, polyethylene, polyurethane, etc.

The amount of the compound of the present invention or a salt thereof to be contained in such a dosage unit form varies depending on the condition of the subject patient or on the dosage form. The desirable amount in one dosage unit form is about 0.05 to about 1000 mg in the case of an oral formulation, about 0.01 to about 500 mg in the case of an injection, and about 1 to about 1000 mg in the case of a suppository. The daily dose of the medicine in such a dosage form depends on the condition, body weight, age, gender, and the like of the patient. For example, the daily dose for an adult may be usually about 0.05 to about 5000 mg, and preferably 0.1 to 1000 mg, and is preferably administered at once or in two to four divided doses per day.

Diseases that can be treated by administering a medicine containing the compound of the present invention are, for example, in the case of malignant tumors, head and neck cancer, esophagus cancer, gastric cancer, colon cancer, rectal cancer, liver cancer, gallbladder and bile duct cancer, pancreatic cancer, lung cancer, breast cancer, ovarian cancer, cervical cancer, endometrium cancer, renal cancer, bladder cancer, prostatic cancer, testicular tumors, bone and soft tissue sarcomas, leukemia, malignant lymphoma, multiple myeloma, skin cancer, brain tumors, etc.

The present invention is described below in more detail with reference to Comparative Examples, Examples, Pharmacological Test Examples, and Formulation Examples. However, the present invention is not limited to these examples.

Example 1

4-N-(n-Nonyloxycarbonyl)-2'-cyano-2'-deoxy-3',5'-O-(1,1,3,3-tetraisopropyldisiloxane-1,3-diyl)-1-β-D- arabinofuranosylcytosine (Compound 1a)

3',5'-O-(1,1,3,3-tetraisopropyldisiloxane-1,3-diyl)-2'-cyano-2'-deoxy-1-β-D-arabinofuranosylcytosine (hereinafter referred to as 3',5'-O-TIPDS-CNDAC) (3.0 ml) was dissolved in Pyridine (500 mg). n-Nonyl chloroformate (244 mg) was added thereto under a nitrogen atmosphere, and the mixture was stirred at room temperature for 1.5 hours. The reaction mixture was separated with ethyl acetate and water. The organic layer was washed with brine, and then dried over anhydrous sodium sulfate. Solvent was removed, and the residue was purified by neutral silica gel column chromatography (5% methanol/chloroform) to give Compound 1a (532 mg, 81%) as a white foam.

$^1$H-NMR (DMSO-$d_6$) δ 10.8 (1H, br s), 8.00 (1H, d, J = 7.6 Hz), 7.09 (1H, d, J = 7.6 Hz), 6.19 (1H, d, J = 7.8 Hz), 4.76-4.65 (1H, m), 4.26-3.90 (6H, m), 1.61-1.56 (2H, m), 1.38-1.12 (12H, m), 1.09-0.96 (28H, m), 0.86-0.82 (3H, m); FAB-LRMS (negative) m/z 663 $(M-H)^-$.

4-N-(n-Nonyloxycarbonyl)-2'-cyano-2'-deoxy-1-β-D- arabinofuranosylcytosine (Compound 1)

A mixed solution of a 1.0 M tetrabutylammonium fluoride (hereinafter referred to as TBAF)/THF solution (1.44 ml) and acetic acid (82.2 μl) was added to a THF (2.9 ml) solution of Compound 1a (532 mg) under a nitrogen atmosphere, and the mixture was stirred at room temperature for 10 minutes. The reaction mixture; was purified by neutral silica gel column chromatography (0-5% methanol/chloroform) to give Compound 1 (174 mg, 52%) as a white foam.
$^1$H-NMR(DYSO-$d_6$)δ 10.8 (1H, br s), 8.33 (1H, d, J = 7.6 Hz), 7.09 (1H, d, J = 7. 6 Hz), 6.27-6.24 (1H, m), 6.20 (1H, d, J = 7.0 Hz), 5.22 (1H, t, J = 5.4 Hz), 4.47-4.37 (1H, m), 4.10 (2H, t, J = 6.5 Hz), 3.95-3.71 (3H, m), 3.67-3.59 (1H, m), 1.61-1.54 (2H, m), 1.40-1.21 (12H, m), 0.86-0.82 (3H, m); FAP-LRMS m/z 423 ($MH^+$).
mp 66°C.

Example 2

5-Nonyl chloroformate (Compound 2a)

5-Nonanol (8.69 ml) was added dropwise to a dichloromethane (12.5 ml) solution of triphosgene (4.97 g) at 0 °C under a nitrogen atmosphere. A dichloromethane (2.5 ml) solution of pyridine (4.0 ml) was added dropwise thereto with keeping the internal temperature under 10°C, and the mixture was stirred at room temperature for 2 hours. The reaction mixture was separated with dichloromethane and cold water. The organic layer was washed with brine, and then dried over calcium chloride. Solvent was distilled off, and the residue was purified by distillation under reduced pressure (13 mmHg, 95 to 96 °C) to give Compound 2a (8.68 g, 84%) as a colorless liquid.
$^1$H-NMR($CDCl_3$)δ 4.96 (1H, m), 1.66-1.57 (4H, m), 1.41-1.36 (8H, m), 0.93 (6H, t, J = 7.3 Hz).

4-N-(Nonan-5-yloxycarbonyl)-2'-cyano-2'-deoxy-3',5'-O-(1,1,3,3-tetraisopropyldisiloxane-1,3-diyl)-1-β-D- arabinofuranosylcytosine (Compound 2b)

Using 3',5'-O-TIPDS-CNDAC (650 mg) and 5-nonyl chloroformate (Compound 2a) (407 mg) as raw material compounds, Compound 2b (780 mg, 89%) was obtained as a white foam in the same manner as Compound 1 was obtained.
$^1$H-NMR(DMSO-$d_6$)δ 10.7 (1H, br s), 7.98 (1H, d, J = 7.6 Hz), 7.09 (1H, d, J = 7. 6 Hz), 6.19 (1H, d, J = 7.3 Hz), 4.81-4.72 (2H, m), 4.62-4.15 (2H, m), 3.98-3.90 (2H, m), 1.62-1.43 (4H, m), 1.38-1.16 (8H, m), 1.12-1.00 (28H, m), 0.95-0.84 (6H, m); FAB-LRMS (negative) m/z 663 $(M-H)^-$.

4-N-(Nonan-5-yloxycarbonyl)-2'-cyano-2'-deoxy-1-β-D-arabinofuranosylcytosine (Compound 2)

Using a mixed solution of Compound 2b (780 mg), a 1.0 M TBAF/THF solution (2.35 ml), and acetic acid (0.13 ml), Compound 2 (296 mg, 60%) was obtained as a white foam in the same manner as Compound 1 was obtained.
$^1$H-NMR(DMSO-$d_6$)δ 10.7 (1H, br s), 8.32 (1H, d, J = 7.6 Hz), 7.08 (1H, d, J = 7.6 Hz), 6.26 (1H, br s), 6.19 (1H, d, J = 7.0 Hz), 5.25 (1H, br s), 4.81-4.72 (1H, m), 4.43-4.39 (1H, m), 3.92-3.60 (4H, m), 1.55-1.53 (4H, m), 1.35-1.20 (8H, m), 0.91-0.83 (6H, m); FAB-LRMS (negative) m/z 421 $(M-H)^-$.
mp 83.6 °C.

Example 3

4-Nitrophenyl-(Z)-6-nonen-1-yl carbonate (Compound 3a)

A mixed solution of (Z)-6-nonen-1-ol (5.0 g), pyridine (2.99 ml), and dichloromethane (11.2 ml) was added to a dichloromethane (25.8 ml) solution of 4-nitrophenyl chloroformate (3.54 g) at 0 °C under a nitrogen atmosphere. The mixture was then warmed to room temperature and stirred overnight. Solvent was distilled off under reduced pressure, and the obtained residue was purified by neutral silica gel column chromatography (11% ethyl acetate/hexane). Compound 3a (3.48 g, 64%) was obtained as a colorless liquid.
$^1$H-NMR(DMSO-$d_6$)δ 8.28 (2H, d, J = 9.2 Hz), 7.38 (2H, d, J = 9.2 Hz), 5.44-5.27 (1H, m), 4.29 (2H, t, J = 6.6 Hz), 2.10-1.99 (2H, m), 1.82-1.65 (2H, m), 1.54-1.35 (6H, m), 0.96 (3H, t, J = 7.4 Hz); FAB-LRMS m/z 308 ($MH^+$).

4-N-[(Z)-6-Nonen-1-yloxycarbonyl]-2'-cyano-2'-deoxy-3',5'-O-(1,1,3,3-tetraisopropyldisiloxane-1,3-diyl)-1-β-D- arabinofuranosylcytosine (Compound 3b)

Compound 3a (1.74 g) and 4-(N,N-dimethylamino)pyridine (173 mg) were added to a dichloromethane (4.24

ml) solution of 3',5'-O-TIPDS-CNDAC (700 mg) under a nitrogen atmosphere, and the mixture was stirred at room temperature for three days. n-Nonyl chloroformate (244 mg) was added thereto, and the obtained mixture was stirred at room temperature for 1.5 hours. Solvent was removed under reduced pressure, and the obtained residue was purified by neutral silica gel column chromatography (0-5% methanol/chloroform). Compound 3b (678 g, 72%) was obtained as a white foam.
$^1$H-NMR (DMSO-$d_6$) δ 10.7 (1H, br s), 8.00 (1H, d, J = 7.6 Hz), 7.10 (1H, d, J = 7.6 Hz), 6.19 (1H, d, J = 7.8 Hz), 5.38-5.24 (2H, m), 4.75-4.65 (1H, m), 4.26-4.08 (4H, m), 3.98-3.90 (2H, m), 2.01-1.96 (4H, m), 1.62-1.57 (2H, m), 1.35-1.30 (4H, m), 1.08-0.94 (28H, m), 0.90-0.86 (3H, m); FAB-LRMS (negative) m/z 661 $(M-H)^-$.

4-N-[(Z)-6-Nonen-1-yloxycarbonyl]-2'-cyano-2'-deoxy-1-β-D- arabinofuranosylcytosine (Compound 3)

Using a mixed solution of Compound 3b (678 mg), a 1.0 M TBAF/THF solution (2.04 ml), and acetic acid (0.12 ml), Compound 3 (298 mg, 69%) was obtained as a white foam in the same manner as Compound 1 was obtained.
$^1$H-NMR(DMSO-$d_6$)δ 10.8 (1H, br s), 8.33 (1H, d, J = 7.6 Hz), 7.09 (1H, d, J = 7.6 Hz), 6.25 (1H, d, J = 5.7 Hz), 6.20 (1H, d, J = 7.0 Hz), 5.40-5.21 (2H, m), 4.46-4.39 (1H, m), 4.10 (2H, d, J = 6.5 Hz), 3.92-3.71 (3H, m), 3.66-3.58 (1H, m), 2.04-1.93 (4H, m), 1.62-1.57 (2H, m), 1.38-1.30 (4H, m), 0.90 (3H, t, J = 7.6 Hz); FAB-LRMS m/z 421 $(MH^+)$.
mp 72.4 ˚C.

Example 4

4-Nitrophenyl-(Z)-3-nonen-1-yl carbonate (Compound 4a)

Using (Z)-3-nonen-1-ol (5.0 g), Compound 4a (2.91 g, 54%) was obtained as a colorless liquid in the same manner as Compound 3a was obtained.
$^1$H-NMR($CDCl_3$)δ 8.28 (2H, d, J = 9.2 Hz), 7.38 (2H, d, J = 9.2 Hz), 5.64-5.53 (1H, m), 5.44-5.33 (1H, m), 4.31-4.26 (2H, m), 2.56-2.53 (2H, m), 2.08-2.03 (2H, m), 1.43-1.23 (6H, m), 0.91-0.86 (3H, m); FAB-LRMS m/z 308 $(MH^+)$.

4-N-[(Z)-3-Nonen-1-yloxycarbonyl]-2'-cyano-2'-deaxy-3',5'-O-(1,1,3,3-tetraisopropyldisiloxane-1,3-diyl)-1-β-D-arabinofuranosylcytosine (Compound 4b)

Using 3',5'-O-TIPDS-CNDAC (700 mg) and Compound 4a (1.74 g), Compound 4b (693 mg, 74%) was obtained as a white foam in the same manner as Compound 3a was obtained.
$^1$H-NMR(DMSO-$d_6$) δ 10.8 (1H, br s), 8.00 (1H, d, J = 7.6 Hz), 7.10 (1H, d, J = 7.6 Hz), 6.19 (1H, d, J = 7.3 Hz), 5.52-5.30 (2H, m), 4.80-4.60 (1H, m), 4.26-4.08 (4H, m), 3.96-3.90 (2H, m), 2.40-2.32 (2H, m), 2.03-1.95 (2H, m), 1.34-1.23 (6H, m), 1.14-0.94 (28H, m), 0.84-0.79 (3H, m); FAB-LRMS m/z 663 $(MH^+)$.

4-N-[(Z)-3-Nonen-1-yloxycarbonyl]-2'-cyano-2'-deoxy-1-β-D-arabinofuranosylcytosine (Compound 4)

Using a mixed solution of Compound 4b (693 mg), a 1.0 M TBAF/THF solution (2.09 ml), and acetic acid (0.12 ml), Compound 4 (312 mg, 71%) was obtained as a white foam in the same manner as Compound 1 was obtained.
$^1$H-NMR(CMSO-$d_6$) δ 10.8 (1H, br s), 8.33 (1H, d, J = 7.6 Hz), 7.08 (1H, d, J = 7.6 Hz), 6.25 (1H, d, J = 5.4 Hz), 6.19 (1H, d, J = 7.0 Hz), 5.52-5.43 (1H, m), 5.40-5.31 (1H, m), 5.22 (1H, t, J = 5.1 Hz), 4.46-4.39 (1H, m), 4.10 (2H, t, J = 6.8 Hz), 3.92-3.58 (4H, m), 2.40-2.32 (2H, m), 2.03-1.96 (2H, m), 1.35-1.20 (6H, m), 0.88-0.80 (3H, m); FAB-LRMS m/z 421 $(MH^+)$.
mp 65 ˚C.

Example 5

4-Nitrophenyl-(E)-2-nonen-1-yl carbonate (Compound 5a)

Using (E)-2-nonen-1-ol (5.0 g), Compound 5a (2.04 g, 38%) was obtained as a colorless liquid in the same manner as Compound 3a was obtained.
$^1$H-NMR($CDCl_3$)δ 8.28 (2H, d, J = 9.2 Hz), 7.38 (2H, d, J = 9.2 Hz), 5.97-5.86 (1H, m), 5.70-5.60 (1H, m), 4.73-4.71 (2H, m), 2.14-2.03 (2H, m), 1.43-1.28 (8H, m), 0.91-0.86 (3H, m); FAB-LRMS m/z 308 $(MH^+)$.

4-N-[(E)-2-Nonen-1-yloxycarbonyl]-2'-cyano-2'-deoxy-3',5'-O-(1,1,3,3-tetraisoprnpyldisiloxane-1,3-diyl)-1-β-D-arabinofuranosylcytosine (Compound 5b)

Using 3',5'-O-TIPDS-CNDAC (700 mg) and Compound 5a (1.74 g), Compound 5b (719 mg, 75%) was obtained as a white foam in the same manner as Compound 3a was obtained.
$^1$H-NMR(DMSO-$d_6$) δ 10.8 (1H, br s), 8.00 (1H, d, J = 7.6 Hz), 7.10 (1H, d, J = 7.6 Hz), 6.19 (1H, d, J= 7.8 Hz), 5.70-5.45 (2H, m), 4.69-4.67 (1H, m), 4.26-4.08 (4H, m), 3.96-3.91 (2H, m), 2.10-2.00 (2H, m), 1.45-1.23 (8H, m), 1.11-0.94 (28H, m), 0.86-0.81 (3H, m); FAB-LRMS m/z 663 ($MH^+$).

4-N-[(F)-2-Nonen-1-yloxycarbonyl]-2'-cyano-2'-deoxy-1-β-D-arabinofuranosylcytosine (Compound 5)

Using a mixed solution of Compound 5b (719 mg), a 1.0 M TBAF/THF solution (2.17 ml), and acetic acid (0.12 ml), Compound 5 (227 mg, 50%) was obtained as a white foam in the same manner as Compound 1 was obtained.
$^1$H-NMR(DMSO-$d_6$) δ 10.8 (1H, br s), 8.34 (1H, d, J = 7.6 Hz), 7.09 (1H, d, J = 7.6 Hz), 6.25 (1H, d, J = 5.4 Hz), 6.19 (1H, d, J = 7.3 Hz), 5.69-5.47 (2H, m), 5.22 (1H, t, J = 5.4 Hz), 4.67 (1H, d, J = 6.5 Hz), 4.46-4.39 (1H, m), 3.92-3.71 (3H, m), 3.66-3.58 (1H, m), 2.12-2.05 (2H, m), 1.35-1.24 (8H, m), 0.89-0.82 (3H, m); FAB-LRMS (negative) m/z 419 $(M-H)^-$.
mp 71.1 ˚C.

Example 6

4-Nitrophenyl-8-nonen-1-yl carbonate (Compound 6a)

Using 8-nonen-1-ol (5.0 g), Compound 6a (3.80 g, 70%) was obtained as a. colorless liquid in the same manner as Compound 3a was obtained.
$^1$H-NMR($CDCl_3$)δ 8.28 (2H, d, J = 9.2 Hz), 7.38 (2H, d, J = 9.2 Hz), 5.89-5.74 (1H, m), 5.04-4.92 (2H, m), 4.29 (2H, t, J = 6.8 Hz), 2.09-2.02 (2H, m), 1.82-1.74 (2H, m), 1.45-1.36 (6H, m); FAB-LRMS
m/z 308 ($MH^+$).

4-N-(8-Nonen-1-yloxycarbonyl)-2'-cyano-2'-deoxy-3',5'-O-(1,1,3,3-tetraisopropyldisiloxane-1,3-diyl)-1-β-D-arabinofuranosylcytosine (Compound 6b)

Using 3',5'-O-TIPDS-CNDAC (700 mg) and Compound 6a (1.74 g), Compound 6b (632 mg, 67%) was obtained as a white foam in the same manner as Compound 3a was obtained.
$^1$H-NMR(DMSO-$d_6$)δ 10.8 (1H, br s), 8.00 (1H d, J = 7.6 Hz), 7.10 (1H, d, J = 7.6 Hz), 6.19 (1H, d, J = 7.6 Hz), 5.85-5.70 (1H, m), 5.02-4.89 (2H, m), 4.78-4.68 (1H, m), 4.26-4.08 (4H, m), 3.90-3.80 (2H, m), 2.04-1.90 (2H, m), 1.61-1.56 (2H, m), 1.39-1.20 (6H, m), 1.28-0.82 (28H, m); FAB-LRMS (negative) m/z 661 $(M-H)^-$.

4-N-(8-Nonen-1-yloxycarbonyl)-2'-cyano-2'-deoxy-1-β-D-arabinofuranosylcytosine (Compound 6)

Using a mixed solution of Compound 6b (632 mg), a 1.0 M TBAF/THF solution (1.91 ml), and acetic acid (0.11 ml), Compound 6 (269 mg, 67%) was obtained as a white foam in the same manner as Compound 1 was obtained.
$^1$H-NMR(DMSO-$d_6$)δ 10.8 (1H, br s), 8.33 (1H, d, J = 7.6 Hz), 7.09 (1H, d, J = 7.6 Hz), 6.25 (1H, d, J = 5.7 Hz), 6.20 (1H, d, J = 7.0 Hz), 5.85-5.79 (1H m), 5.76-5.70 (1H, m), 5.24 -5.20 (1H, m), 4.46-4.39 (1H, m), 4.10 (1H, t, J = 6.8 Hz), 3.92-3.72 (3H, m), 3.66-3.58 (1H, m), 2.04-1.96 (2H, m), 1.65-1.50 (2H, m), 1.40-1.20 (8H, m); FAB-LRMS m/z 421 ($MH^+$).
mp 72.7 ˚C.

Example 7

4-Nitrophenyl-(Z)-2-nonen-1-yl carbonate (Compound 7a)

Using (Z)-2-nonen-1-ol (5.0 g), Compound 7a (3.8 g, 71%) was obtained as a colorless liquid in the same manner as Compound 3a was obtained.
$^1$H-NMR($CDCl_3$)δ 8.28 (2H, d, J = 9.2 Hz), 7.38 (2H, d, J = 9.2 Hz), 5.83-5.73 (1H, m), 5.67-5.57 (1H, m), 4.85-4.82 (2H, m), 2.19-2.11 (2H, m), 1.48-1.23 (6H, m), 0.91-0.86 (3H, m); FAB-LRMS m/z 308 ($MH^+$).
mp 62 ˚C

4-N-[(Z)-2-Nonen-1-yloxycarbonyl]-2'-cyano-2'-deoxy-3',5'-O-(1,1,3,3-tetraisopropyldisiloxane-1,3-diyl)-1-β-D-arabinofuranosylcytosine (Compound 7b)

Using 3'5'-O-TIPDS-CNDAC (700 mg) and Compound 7a (1.74 g), Compound 7b (852 mg, 91%) was obtained as a white foam in the same manner as Compound 3a was obtained.
$^1$H-NMR(DMSO-$d_6$)δ 10.8 (1H, br s), 8.00 (1H, d, J = 7.6 Hz), 7.10 (1H, d, J = 7.6 Hz), 6.19 (1H, d, J = 7.3 Hz), 5.87-5.76 (1H, m), 5.61-5.51 (1H, m), 4.75-4.65 (1H, m), 4.58-4.56 (2H, m), 4.26-4.15 (2H, m), 3.97-3.92 (2H, m), 2.20-1.98 (2H, m), 1.32-1.23 (8H, m), 1.12-0.99 (28H, m), 0.90-0.81 (3H, m); FAB-LRMS m/z 663 ($MH^+$).

4-N-[(Z)-2-Nonen-1-yloxycarbonyl]-2'-cyano-2'-deoxy-1-β-D-arabinofuranosylcytosine (Compound 7)

Using a mixed solution of Compound 7b (852 mg), a 1.0 M TBAF/THF solution (2.75 ml), and acetic acid (0.15 ml), Compound 7 (234 mg, 43%) was obtained as a white foam in the same manner as Compound 1 was obtained.
$^1$H-NMR(DMSO-$d_6$)δ 10.8 (1H, br s), 8.34 (1H, d, J = 7.6 Hz), 7.09 (1H, d, J = 7.6 Hz), 6.25 (1H, d, J = 5.7 Hz), 6.19 (1H, d, J = 7.3 Hz), 5.87-5.76 (1H, m), 5.61-5.51 (1H, m), 5.22 (1H, t, J = 5.4 Hz), 4.57 (1H, d, J = 5.9 Hz), 4.46-4.39 (1H, m), 3.92-3.72 (3H, m), 3.66-3.59 (1H, m), 2.05-1.98 (2H, m), 1.30-1.20 (8H, m), 0.90-0.81 (3H, m); FAB-LRMS (negative) m/z 419 $(M-H)^-$.

Example 8

4-N-Acetyl-5'-O-(n-hexyloxyoarborayl)-3'-O-triisopropylsilyl-2'-cyano-2'-deoxy-1-β-D-arabinofuranosylcytosine (Compound 8a)

n-Hexyl chloroformate (980 μl) was added to a pyridine (3.0 ml) solution of 4-N-acetyl-3'-O-triisopropylsilyl-2'-cyano-2'-deoxy-1-β-D-arabinofuranosylcytosine (hereinafter referred to as 4-N-acetyl-3'-O-TIPS-CNDAC) (450 mg) under a nitrogen atmosphere, and the mixture was stirred at room temperature overnight. The reaction mixture was separated with ethyl acetate and water. The organic layer was washed with brine, and then dried over anhydrous sodium sulfate. Solvent was distilled off, and the residue was purified by neutral silica gel column chromatography (33% ethyl acetate/hexane) to give Compound 8a (462 mg, 80%) as a white foam.
$^1$H-NMR(DMSO-$d_6$)δ 11.0 (1H, s), 8.09 (1H, d, J = 7.3 Hz), 7.27 (1H, d, J = 7.3 Hz), 6.21 (1H, d, J = 7.3 Hz), 4.80 (1H, m), 4.42 (2H, m), 4.07 (4H, m), 2.11 (3H, s), 1.57 (2H, m), 1.25 (6H, m), 1.12-1.05 (21H, m), 0.83 (3H, m); FAB-LRMS m/z (negative) 577 $(M-H)^-$.

4-N-Acetyl-5'-O-(n-hexyloxycarbonyl)-2'-cyano-2'-deoxy-1-β-D-arabinofuranosylcytosine (8b)

A mixed solution of a 1.0 M TBAF/THF solution (874 μl) and acetic acid (50.6 μl) was added to a THF (3.9 ml) solution of Compound 8a (462 mg), and the mixture was stirred at room temperature for 1 hour. The reaction mixture was purified by neutral silica gel column chromatography (0-5% methanol/chloroform) to give Compound 8b (220 mg, 65%) as a white foam.
$^1$H-NMR(DMSO-$d_6$)δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.49-4.34 (3H, m), 4.00-3.77 (4H, m), 2.16 (3H, s), 1.58 (2H, m), 1.33 (6H, m), 0.81 (3H, m); FAB-LPMS m/z 423 ($MH^+$).

5'-O-(n-Hexyloxycarbonyl)-2'-cyano-2'-deoxy-1-β-D-arabinofuranosylcytosine (Compound 8)

Compound 8b (220 mg) was dissolved in a 10% hydrochloric acid/methanol solution (5.2 ml), and stirred at room temperature for 5 hours. The reaction solution was concentrated and separated with ethyl acetate and a saturated aqueous sodium hydrogen carbonate solution. The organic layer was washed twice with a saturated aqueous sodium hydrogen carbonate solution and once with brine, and then dried over anhydrous sodium sulfate. Solvent was distilled off, and hexane was added thereto. The mixture was solidified by ultrasonic waves. The precipitate was collected by filtration to give Compound 8 (120 mg, 61%) as a white powder.
$^1$H-NMR(DMSO-$d_6$)δ 7.59 (1H, d, J = 7.3 Hz), 7.36 (2H, br d), 6.34 (1H, m), 6.17 (1H, d, J = 7.3 Hz), 5.77 (1H, d, J = 7.8 Hz), 4.39 (2H, m), 4.32 (1H, m), 4.08 (2H, t, 6.6 Hz), 3.92 (1H, m), 3.83 (1H, t, J = 7.6 Hz), 1.58 (2H, m), 1.26 (6H, m), 0.84 (3H, m); FAB-LRMS m/z (negative) 379 $(M-H)^-$.
Anal. Calcd for $C_{17}H_{24}N_4O_6$. C, 53.68; H, 6.36; N, 14.73. Found: C, 53.55; H, 6.29; N, 14.57;
mp 154.1 ˚C.

Example 9

4-N-Acetyl-5'-O-(n-heptyloxycarbonyl)-3'-O-dimethylthexylsilyl-2'-cyano-2'-deoxy-1-β-D-arabinofuranosylcytosine (Compound 9a)

Using 4-N-acetyl-3'-O-dimethylthexylsilyl-2'-cyano-2'-deoxy-1-β-D-arabinofuranosylcytosine (hereinafter referred to as 4-N-acetyl-3'-O-TDS-CNDAC) (300 mg, 0.69 mmol) and n-heptyl chloroformate (490 μl), Compound 9a (360 mg, 90%) was obtained as a white foam in the same manner as Compound 8a was obtained.
$^1$H-NMR(DMSO-$d_6$)δ 11.0 (1H, s), 8.08 (1H, d, J = 7.3 Hz), 7.28 (1H, d, J = 7.3 Hz), 6.26 (1H, d, J = 7.3 Hz), 4.67 (1H, m), 4.37 (2H, m), 4.07 (4H, m), 2.11 (3H, s), 1.57 (3H, m), 1.26 (8H, m), 0.83 (15H, m), 0.19 (6H, m); FAB-LRMS m/z 579 ($MH^+$).

4-N-Acetyl-5'-O-(n-heptyloxycarbonyl)-2'-cyano-2'-deoxy-1-β-D-arabinofuranosylcytosine (Compound 9b)

Using a mixed solution of Compound 9a (360 mg), a 1.0 M TBAF/THF solution (746 μl), and acetic acid (43 μl), Compound 9b (150 mg, 55%) was obtained as a white foam in the same manner as Compound 8b was obtained.
$^1$H-NMR(DMSO-$d_6$)δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.49-4.34 (3H, m), 4.00-3.77 (4H, m), 2.16 (3H, s), 1.58 (2H, m), 1.33 (8H, m), 0.81 (3H, m); FAB-LRMS m/z 437 ($MH^+$) .

5'-O-(n-Heptyloxycarbonyl)-2'-cyano-2'-deoxy-1-β-D-arabinofuranosylcytosine (Compound 9)

Using Compound 9b (150 mg) and a 10% hydrochloric acid/methanol solution (3.4 ml), Compound 9 (96 mg, 72%) was obtained as a white powder in the same manner as Compound 8 was obtained.
$^1$H-NMR(DMSO-$d_6$)δ 7.60 (1H, d, J = 7.6 Hz), 7.40 (2H, br d), 6.35 (1H, d, J = 5.6 Hz), 6.18 (1H, d, J = 7.6 Hz), 5.78 (1H, d, J = 7. 6 Hz), 4.40 (1H, m), 4.31 (1H, dd, J = 6.8 Hz, 12.0 Hz), 4.08 (2H, t, J = 6.6 Hz), 3.93 (1H, m), 3.83 (1H, m), 1.58 (2H, m), 1.25 (8H, m), 0.84 (3H, t, J= 6.6 Hz); FAB-LRMS m/z 395 ($MH^+$).
Anal. Calcd for $C_{18}H_{26}N_4O_6$: C, 54.81; H, 6.64; N, 14.20. Found: C, 54.58; H, 6.68; N, 14.09;
mp 152.2 °C.

Example 10

4-N-Acetyl-5'-O-(n-octyloxycarbonyl)-3'-O-triisopropylsilyl-2'-cyano-2'-deoxy-1-β-D-arabinofuranosylcytosine (Compound 10a)

Using 4-N-acetyl-3'-O-TIPS-CNDAC (300 mg) and n-octyl chloroformate (157 μl). Compound 10a (583 mg, 70%) was obtained as a white foam in the same manner as Compound 8a was obtained.
$^1$H-NMR(DMSO-$d_6$) δ 11.0 (1H, s), 8.08 (1H, d, J = 7.8 Hz), 7.28 (1H, d, J = 7.8 Hz), 6.27 (1H, d, J = 7.8 Hz), 4.63 (2H, m), 4.38 (2H, m), 4.05 (2H, m), 2.11 (3H, s), 1.57 (5H, m), 1.25 (8H, m), 0.84 (18H, m), 0.19 (6H, m); FAB-LRMS m/z 607 ($MH^+$) .

4-N-Acetyl-5'-O-(n-octyloxycarborayl) -2'-cyano-2'-deoxy-1-β-D-arabinofuranosylcytosine (Compound 10b)

Using a mixed solution of Compound 10a (580 mg), a 1.0 M TBAF/THF solution (1.05 ml), and acetic acid (60 μl), Compound 10b (193 mg, 45%) was obtained as a white foam in the same manner as Compound 8b was obtained.
$^1$H-NMR(DMSO-$d_6$) δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.49-4.34 (3H, m), 4.00-3.77 (4H, m), 2.16 (3H, s), 1.58 (2H, m), 1.33 (10H, m), 0.81 (3H, m) .

5'-O-(n-Octyloxycarbonyl)-2'-cyano-2'-deoxy-1-β-D-arabinofuranosylcytosine (Compound 10)

Using Compound 10b (2.4 g) and a 10% hydrochloric acid/methanol solution (50 ml), Compound 10 (1.95 g, 90%) was obtained as a white powder in the same manner as Compound 8 was obtained.
$^1$H-NMR(DMSO-$d_6$)δ 7.57 (1H, d, J = 7.6 Hz), 7.29 (2H, br d), 6.34 (1H, d, J = 5.6 Hz), 6.17 (1H, d, J = 7.6 Hz), 5.78 (1H, d, J = 7.6 Hz), 4.40 (1H, m), 4.31 (1H, dd, J = 6.8 Hz, 12.0 Hz), 4.08 (2H, t, J = 6.6 Hz), 3.93 (1H, m), 3.83 (1H, m), 1.58 (2H, m), 1.25 (8H, m), 0.84 (3H, t, J = 6.6 Hz); FAB-LRMS m/z 409 ($MH^+$).
Anal. Calcd for $C_{19}H_{28}N_4O_6$: C, 55.87; H, 6.91; N, 13.72. Found: C, 55.71; H, 6.92; N, 13.62
mp 143.1 °C.

Example 11

4-N-Acetyl-5'-O-(nonan-5-yloxycarbonyl)-3'-O-dimethylthexylsilyl-2'-cyano-2'-deoxy-1-β-D-arabinofuranosylcytosine (Compound 11a)

Using 4-N-acetyl-3'-O-TDS-CNDAC (300 mg) and Compound 2a (713 mg), Compound 11b (392 mg, 94%) was obtained as a white foam in the same manner as Compound 8a was obtained.
$^1$H-NMR(DMSO-$d_6$)δ 11.0 (1H, s), 8.08 (1H, d, J = 7.6 Hz), 7.30 (1H, d, J = 7.6 Hz), 6.28 (1H, d, J = 7.6 Hz), 4.83 (1H, m), 4.65 (1H, m), 4.40 (2H, m), 4.07 (2H,m), 2.13 (3H, s), 1.63-1.34 (12H, m), 0.85 (18H, m), 0.19 (6H, m); FAB-LRMS m/z 607 ($MH^+$).

4-N-Acetyl-5'-O-(nonan-5-yloxycarbonyl)-2'-cyano-2'-deoxy-1-β-D-arabinofuranosylcytosine (Compound 11b)

Using a mixed solution of Compound 11a (390 mg), a 1.0 M TBAF/THF solution (704 μl), and acetic acid (40 μl), Compound 11b (200 mg, 67%) was obtained as a white foam in the same manner as Compound 8b was obtained.
$^1$H-NMR(DMSO-$d_6$)δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.49-4.34 (3H, m), 4.00-3.77 (4H, m), 2.16 (3H, s), 1.51 (4H, m), 1.24 (8H, m), 0.84 (6H, m); FAB-LRMS m/z (negative) 449 $(M-H)^-$.

5'-O-(Nonan-5-yloxycarbonyl) -2'-cyano-2'-deoxy-1-β-D-arabinofuranosylcytosine (Compound 11)

Using Compound 11b (200 mg) and a 10% hydrochloric acid/methanol solution (4.3 ml), Compound 11 (143 mg, 79%) was obtained as a white powder in the same manner as Compound 8 was obtained.
$^1$H-NMR(DMSO-$d_6$)δ 7.57 (1H, d, J = 7.3 Hz), 7.30 (2H, br d), 6.34 (1H, d, J = 5.6 Hz), 6.18 (1H, d, J = 7.3 Hz), 5.75 (1H, d, J = 7.3 Hz), 4.61 (1H, m), 4.41 (2H, m), 4.31 (1H, dd, 6.6 Hz, 12.2 Hz), 3.93 (1H, m), 3.83 (1H, t, J = 7.6 Hz), 1.51 (4H, m), 1.24 (8H, m), 0.84 (6H, m); FAB-LRMS m/z 423 ($MH^+$).
Anal. Calcd for $C_{20}H_{30}N_4O_6$: C, 56.86; H, 7.16; N, 13.26. Found: C, 56.55; H, 7.14; N, 13.24;
167.2 ˚C.

Example 12

4-N-Acetyl-5'-O-(n-nonyloxycarbonyl)-3'-O-triisopropylsilyl-2'-cyano-2'-deoxy-1-β-D-arabinofuranosylcytosine (Compound 12a)

Using 4-N-acetyl-3'-O-TIPS-CNDAC (190 mg) and n-nonyl chloroformate (180 μl), Compound 12a (250 mg, 96%) was obtained as a white foam in the same manner as Compound 8a was obtained.
$^1$H-NMR(DMSO-$d_6$)δ 10.8 (1H, s), 8.09 (1H, d, J = 7.6 Hz), 7.27 (1H, d, J = 7.6 Hz), 6.21 (1H, d, J = 7.6 Hz), 4.79 (1H, m), 4.41 (2H,m) 4.15-4.02 (4H, m), 2.11 (3H, s), 1.57-1.04 (2H, m), 0.84 (3H, t, J = 6.6 Hz); FAB-LRMS m/z 621 ($MH^+$).

4-N-Acetyl-5'-O-(n-nonyloxycarbonyl)-2'-cyano-2'-dsoxy-1-β-D-arabinofuranosylcytosine (Compound 12b)

Using a mixed solution of Compound 12a (250 mg), a 1.0 M TBAF/THF solution (0.44 ml), and acetic acid (25 μl), Compound 12b (158 mg, 63%) was obtained as a white foam in the same manner as Compound 8b was obtained.
$^1$H-NMR(DMSO-$d_6$)δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.49-4.34 (3H, m), 4.00-3.77 (4H, m), 2.16 (3H, s), 1.58 (2H, m), 1.33 (12H, m), 0.81 (3H, m).

5'-O-(n-Nonyloxycarbonyl) -2'-cyano-2'-deoxy-1-β-D-arabinofuranosylcytosine (Compound 12)

Using Compound 12b (158 mg) and a 10% hydrochloric acid/methanol solution (2.5 ml), Compound 12 (72 mg, 68%) was obtained as a white powder in the same manner as Compound 8 was obtained.
$^1$H-NMR(DMSO-$d_6$)δ 7.57 (1H, d, J = 7.3 Hz), 7.31, 7.27 (each 1H, each br s), 6.34 (1H, d, J = 5. Hz), 6.17 (1H, d, J = 7.3 Hz), 5.76 (1H, d, J = 7.3 Hz), 4.39 (1H, m), 4.31 (1H, m), 4.08 (2H, t, J = 6.3 Hz), 3.92 (1H, m), 3.82 (1H, m), 1.58 (2H, m), 1.25 (12H, m), 0.83 (3H, m); FAB-LRMS m/z 409 ($MH^+$) .
FAB-LRMS m/z 424 ($MH^+$).
mp 143.1 ˚C.

Example 13

4-Heptyl chloroformate (Compound 13a)

Using triphosgene (4.97 g) and 4-heptanol (7.0 ml), Compound 13a (6.85 g, 77%) was obtained as a colorless liquid after distillation under reduced pressure (13 mmHg, 69 to 70 ˚C) in the same manner as compound 2a was obtained. $^{1}$H-NMR($CDCl_3$)δ 4.96 (1H, m), 1.74-1.54 (4H, m), 1.50-1.29 (4H, m), 0.94 (6H, m).

4-N-Acetyl-5'-O-(heptan-4-yloxycarbonyl)-3'-O-dimethylthexylsilyl-2'-cyano-2'-deoxy-1-β-D-arabinofuranosylcytosine (Compound 13b)

Using 4-N-acetyl-3'-O-TDS-CNDAC (300 mg) and Compound 13a (493 mg), Compound 13b (368 mg, 92%) was obtained as a white foam in the same manner as Compound 8a was obtained.
$^{1}$H-NMR(DMSO-$d_6$)δ 11.0 (1H, s), 8.08 (1H, d, J = 7.8 Hz), 7.28 (1H, d, J = 7.8 Hz), 6.26 (1H, d, J = 7.8 Hz), 4.66 (2H, m), 4.38 (2H, m) 4.04 (2H, m), 2.11 (3H, s), 1.59-1.48 (5H, m), 1.29 (4H,m), 0.86 (18H, m), 0.19, 0.16 (each 3H, each s); FAB-LRMS m/z 579 ($MH^+$).

4-N-Acetyl-5'-O-(heptan-4-yloxycarbonyl)-2'-cyano-2'-deoxy-1-β-D-arabinofuranosylcytosine (Compound 13c)

Using a mixed solution of Compound 13b (360 mg), a 1.0 M TBAF/THF solution (0.68 ml), and acetic acid (39 μl), Compound 13c (200 mg, 74%) was obtained as a white foam in the same manner as Compound 8b was obtained.
$^{1}$H-NMR(DMSO-$d_6$)δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.49-4.34 (3H, m), 4.00-3.77 (4H, m), 2.16 (3H, s), 1.51 (4H, m), 1.27 (4H, m), 0.86 (6H, m).

5'-O-(Heptan-4-yloxycarbonyl) -2'-cyano-2'-deoxy-1-β-D-arabinofuranosylcytosine (Compound 13)

Using Compound 13c (200 mg) and a 10% hydrochloric acid/methanol solution (4.6 ml), Compound 13 (130 mg, 72%) was obtained as a white powder in the same manner as Compound 8 was obtained.
$^{1}$H-NMR(DMSO-$d_6$)δ 7.60 (1H, d, J = 7.3 Hz), 7.51, 7.37 (each 1H, br s), 6.36 (1H, d, J = 5.6 Hz), 6.18 (1H, d, J = 7.3 Hz), 5.78 (1H, d, J = 7.3 Hz), 4.65 (1H, t, J = 6.3 Hz), 4.42 (2H, m), 4.30 (1H, dd, J = 6.3 Hz, 12.0 Hz), 3.93 (1H, m), 3.84 (1H, t, J = 7.6 Hz), 1.51 (4H, m), 1.27 (4H, m), 0.86 (6H, m); FAB-LRMS m/z 397($MH^+$). mp 164.2 ˚C.

Example 14

2,6-Dimethylheptan-4-ylchloroformate (Compound 14a)

Using triphosgene (4.97 g) and 2,6-dimethyl-4-heptanol (8.9 ml), Compound 14a (8.85 g, 86%) was obtained as a colorless liquid after distillation under reduced pressure (19 mmHg, 89 to 90 ˚C) in the same manner as cCompound 2a was obtained.
$^{1}$H-NMR($CDCl_3$)δ 4.96 (1H, m), 1.50 (4H, m), 1.34 (2H, m), 0.86 (12H, m).

4-N-Acetyl-5'-O-(2,6-dimethylheptan-4-yloxycarbonyl)-3'-O-dimethylthexylsilyl-2'-cyano-2'-deoxy-1-β-D-arabinofura no s ylc yt o sine (Compound 14 b)

Using 4-N-acetyl-3'-O-TDS-CNDAC (300 mg) and Compound 14a (1.0 ml), Compound 14b (408 mg, 97%) was obtained as a white foam in the same manner as Compound 8a was obtained.
$^{1}$H-NMR(DMSO-$d_6$)δ 11.0 (1H, s), 8.09 (1H, d, J = 7.6 Hz), 7.28 (1H, d, J = 7.6 Hz), 6.24 (1H, d, J = 7.6 Hz), 4.79 (1H, m), 4.40 (2H, m), 4.31 (1H, dd, J = 6.3 Hz, 12.2 Hz), 3.92 (1H, m), 3.83 (1H, t, J = 7.6 Hz), 2.16 (3H, s), 1.50 (5H, m), 1.34 (2H, m), 0.86 (24H, m), 0.19, 0.16 (each 3H, each s)

4-N-Acetyl-5'-O-(2,6-dimethylheptan-4-yloxycarbonyl)-2'-cyano-2'-deoxy-1-β-D-arabinofuranosylcytosine (Compound 14c)

Using a mixed solution of Compound 14b (408 mg), a 1.0 M TBAF/THF solution (0.74 ml), and acetic acid (42 μl), Compound 14c (135 mg, 43%) was obtained as a white foam in the same manner as Compound 8b was obtained.
$^{1}$H-NMR(DMSO-$d_6$)δ 11.0 (1H, s), 8.09 (1H, d, J = 7.6 Hz), 7.28 (1H, d, J = 7.6 Hz), 6.46 (1H, bs), 6.24 (1H, d, J = 7.6 Hz), 4.79 (1H, m), 4.40 (2H, m), 4.31 (1H, dd, J = 6.3 Hz, 12.2 Hz), 3.92 (1H, m), 3.83 (1H, t, J = 7.6 Hz), 2.16 (3H, s), 1.50 (4H, m), 1.34 (2H, m), 0.86 (12H, m).

5'-O-(2,6-Dimethylheptan-4-yloxycarbonyl)-2'-cyano-2'-deoxy-1-β-D-arabinofuranosylcytosine (Compound 14)

Using Compound 14c (135 mg) and a 10% hydrochloric acid/methanol solution (2.9 ml), Compound 14 (55 mg, 45%) was obtained as a white powder in the same manner as Compound 8 was obtained.
$^1$H-NMR (DMSO-$d_6$)δ 7.56 (1H, d, J = 7.6 Hz), 7.33, 7.28 (each 1H, br s), 6.36 (1H, d, J = 5.6 Hz), 6.18 (1H, d, J = 7.6 Hz), 5.75 (1H, d, J = 7.6 Hz), 4.79 (1H, m), 4.40 (2H, m), 4.31 (1H, dd, J = 6.3 Hz, 12.2 Hz), 3.92 (1H, m), 3.83 (1H, t, J = 7.6 Hz), 1.50 (4H, m), 1.34 (2H, m), 0.86 (12H, m); FAB-LRMS m/z 423(MH$^+$).
mp 182.7 °C (decomp.).

The following Tables 1 to 3 show the structural formulae of Compounds 1 to 14 obtained in the above Examples.

[Table 1]

| Compound | Structural Formula |
|---|---|
| 1 | 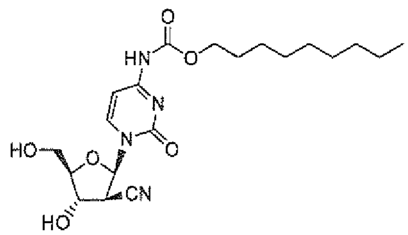 |
| 2 | 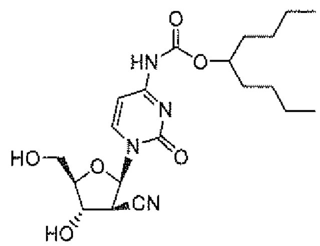 |
| 3 | 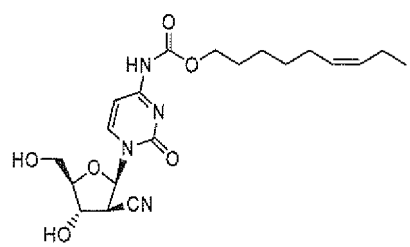 |
| 4 | 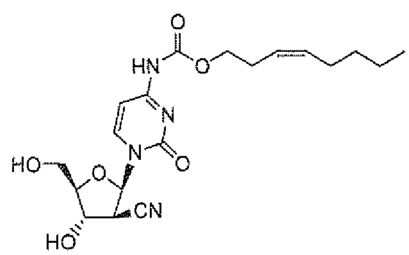 |

(continued)

| Compound | Structural Formula |
|---|---|
| 5 | 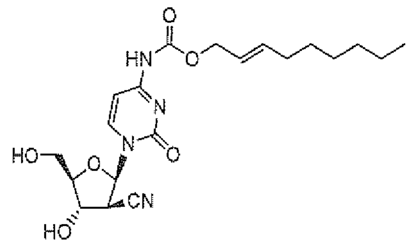 |

[Table 2]

| | |
|---|---|
| 6 | 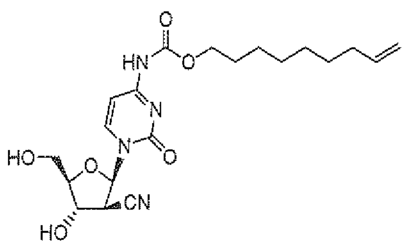 |
| 7 | 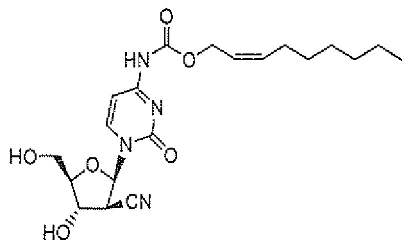 |
| 8 | 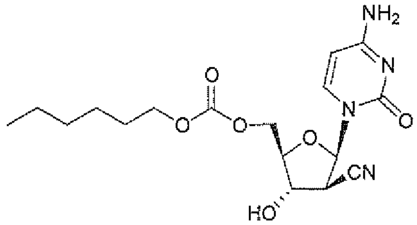 |
| 9 | 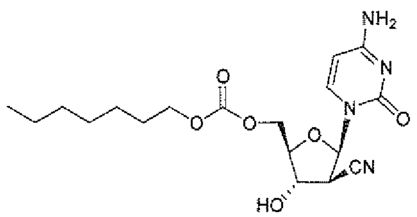 |

[Table 3]

| | |
|---|---|
| 10 | 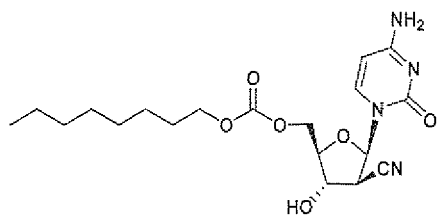 |
| 11 | 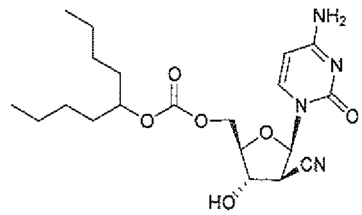 |
| 12 | 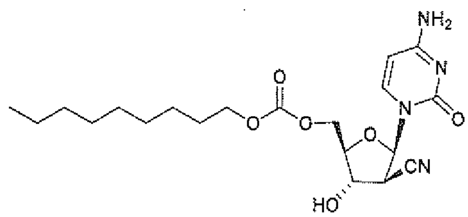 |
| 13 | 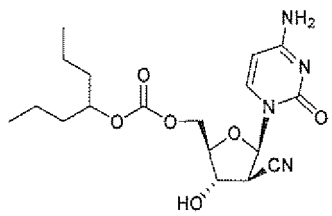 |
| 14 | 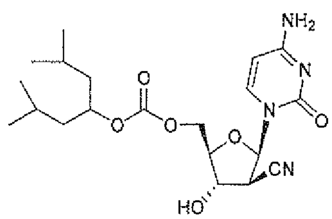 |

Comparative Example 1

4-N-(n-Decyloxycarbonyl)-2'-cyano-2'-deoxy-3',5'-O-(1,1,3,3-tetraisopropyldisiloxane-1,3-diyl)-1-β-D-arabinofuranosylcytosine (Compound 15a)

Using 3',5'-O-TIPDS-CNDAC (500 mg) and n-decyl chloroformate (282 mg), Compound 15a (504 mg, 76%) was obtained as a white foam in the same manner as Compound 1a was obtained.
$^1$H-NMR(DMSO-$d_6$)δ 10.8 (1H, br s), 8.00 (1H, d, J = 7.6 Hz), 7.10 (1H, d, J = 7.6 Hz), 6.19 (1H, d, J = 7.8 Hz), 4.72-4.70 (1H, m), 4.30-3.90 (6H, m), 1.59-1.56 (2H, m), 1.38-1.23 (14H, m), 1.15-1.03 (28H, m), 0.95-0.81 (3H, m); FAB-LRMS m/z 679 (MH$^+$).

4-N-(n-Decyloxycarbonyl)-2'-cyano-2'-deoxy-1-β-D-arabinofuranosylcytosine (Compound 15)

Using a mixed solution of Compound 15a (503 mg), a 1.0 M TBAF/THF solution (1.48 μl), and acetic acid (84.9 μl), Compound 15 (323 mg, 46%) was obtained as a white foam in the same manner as Compound 1 was obtained. $^1$H-NMR(DMSO-$d_6$)δ 10.8 (1H, br s), 8.00 (1H, d, J = 7.6 Hz), 7.10 (1H, d, J = 7.6 Hz), 6.19 (1H, d, J = 7.8 Hz), 4.72-4.70 (1H, br s), 4.30-3.90 (6H, m), 1.59-1.56 (2H, m), 1.38-1.23 (14H, m), 1.15-1.03 (28H, m), 0.95-0.81 (3H, m); FAB-LRMS m/z 437 ($MH^+$).
mp 97 ˚C.

Comparative Example 2

4-N-(n-Octyloxycarbonyl)-2'-cyano-2'-deoxy-1-β-D-arabinofuranosylcytosine (Compound 16)

Trimethylsilyl chloride (0.68 ml) was added to a pyridine (3.5 ml) solution of CNDAC hydrochloride (500 mg), and the obtained mixture was stirred at room temperature for 3 hours. n-Octyl chloroformate (500 mg) was added to the reaction mixture, and the obtained mixture was stirred at room temperature for another 30 minutes. The reaction mixture was separated with ethyl acetate and water . The organic layer was washed with brine, and then dried over anhydrous sodium sulfate. Solvent was distilled off, and the obtained residue was dissolved in THF. A mixed solution of a 1.0 M TBAF/THF solution (3.98 ml) and acetic acid (230 μl) was added thereto, and the mixture was stirred for 10 minutes. Solvent was distilled off, and the residue was purified by neutral silica gel column chromatography (10% methanol/chloroform) to give Compound 16 (537 mg, 76%) as a white foam.
$^1$H-NMR(DMSO-$d_6$)δ 10.8 (1H, br s), 8.34 (1H, d, J = 7.6 Hz), 7.10 (1H, d, J = 7.6 Hz), 6.27 (1H, br s), 6.21 (1H, d, J = 7.1 Hz), 5.24 (1H, br s), 4.43 (1H, m), 4.11 (2H, t, J = 6.3Hz), 3.91 (1H, m), 3.83 (1H, m), 3.66 (1H, m), 1.61 (2H, m), 1.31 (4H, m), 0.88 (3H, m); FAB-LRMS m/z 409 ($MH^+$).
mp 67.5 ˚C

Comparative Example 3

4-N-Acetyl-5'-O-(4-chlorophenyloxycarbonyl)-3'-O-dimethylthexylsilyl-2'-cyano-2'-deoxy-1-β-D-arabinofuranosylcytosine (Compound 17a)

Using 4-N-acetyl-3'-O-TDS-CNDAC (1.0 g) and 4-chlorophenyl chloroformate (1.15 ml), Compound 17a (670 mg, 50%) was obtained as an orange foam in the same manner as Compound 8a was obtained.
$^1$H-NMR(DMSO-$d_6$)δ 11.0 (1H, s), 8.07 (1H, d, J = 7.6 Hz), 7.60 (1H, d, J = 7.3 Hz), 7.27 (3H,m), 6.42 (1H, d, J = 5.6 Hz), 6.23 (1H, d, J = 7.6 Hz), 4.56-4.43 (3H, m), 4.03 (1H, m), 3.85 (1H, m), 2.16 (3H, s), 1.59 (1H, m), 0.86 (12H,m), 0.21, 0.18 (each 3H, each s).

4-N-Acetyl-5'-O-(4-chlorophenyloxycarbonyl)-2'-cyano-2'-deoxy-1-β-D-arabinofuranosylcytosine (Compound 17b)

Using a mixed solution of Compound 17a (500 mg), a 1.0 M TBAF/THF solution (1 ml), and acetic acid (61 μl), Compound 17b (240 mg, 63%) was obtained as an orange foam in the same manner as Compound 8b was obtained.
$^1$H-NMR(DMSO-$d_6$)δ 11.0 (1H, s), 8.07 (1H, d, J = 7.6 Hz), 7.60 (1H, d, J = 7.3 Hz), 7.27 (3H,m), 6.42 (1H, d, J = 5.6 Hz), 6.23 (1H, d, J = 7.6 Hz), 4.56-4.43 (3H, m), 4.03 (1H, m), 3.85 (1H, m), 2.16 (3H, s).

5'-O-(4-Chlorophenyloxycarbonyl)-2'-cyaro-2'-deoxy-1-β-D-arabinofuranosylcytosine (Compound 17)

Using Compound 17b (240 mg) and a 10% hydrochloric acid/methanol solution (5.5 ml), Compound 17 (154 mg, 71%) was obtained as a white powder in the same manner as Compound 8 was obtained.
$^1$H-NMR(DMSO-$d_6$)δ 7.60 (1H, d, J = 7.3 Hz), 7.30 (4H,m), 6.39 (1H, d, J = 5.4 Hz), 6.19 (1H, d, J = 7.8 Hz), 5.77 (1H, d, J = 7.3 Hz), 4.56-4.43 (3H, m), 4.03 (1H, m), 3.85 (1H, m); FAB-LRMS m/z 405 ($MH^+$).
mp 113.0 ˚C .

The following Table 4 shows the structural formulae of Compounds 15 to 17 obtained in the above Comparative Examples.

[Table 4]

| Compound | Structural Formula |
|---|---|
| 15 | 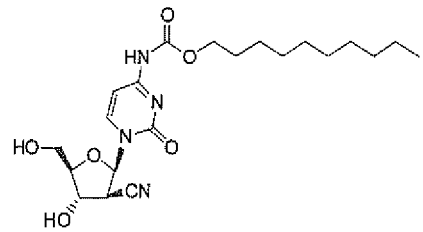 |
| 16 | 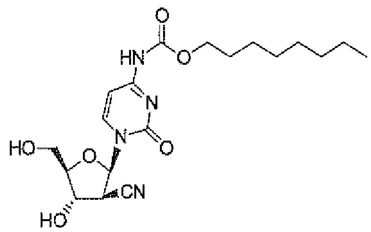 |
| 17 | 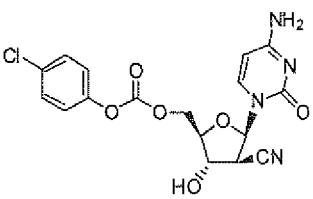 |

[Test Example 1]

Antitumor Test using Tumor-Subcutaneously-Implanted Nude Mouse System upon Oral Administration

Human colon cancer strain KM20C subcutaneously subcultured in a BALB/cA Jcl-nu mouse (product of Clea Japan Inc.) was prepared into fragments of 2 mm × 2 mm, and subcutaneously implanted into the backs of 6-week-old BALB/cA Jcl-nu mice. When the average tumor volume exceeded 100 $mm^3$, the length of each tumor was measured along the major axis and minor axis, and the tumor volume was calculated using the following equation. The mice were then divided into several groups to reduce variations in tumor volume among the groups (5 or 6 mice per group).

$$\text{(Equation 1)} \quad Vt = 1/2(Vl) \times (Vs)^2,$$

wherein Vt represents a tumor volume, Vl represents the length along the tumor major axis, and Vs represents the length along the tumor minor axis.

Test compounds were each dissolved or suspended in a 0.5% hydroxypropylmethylcellulose solution prepared with 100 mM citrate buffer (pH 5.0), and, from the day after the day of grouping, orally administered to the mice once per day for 14 days. The dose was set to be equimolar to 18 mg of CNDAC/kg of body weight per day.

On the 29th day after the day of grouping, with respect to the mice in each group, the longest diameter and shortest diameter of each of the subcutaneously implanted tumors were measured. The relative tumor volume (RTV) and the tumor growth inhibition rate (IR) were calculated using the following equation to evaluate antitumor effects. The test results are shown in Table 5.

$$\text{(Equation 2)} \quad RTV = Vt1/Vt2,$$

wherein RTV represents a relative tumor volume, Vt1 represents the tumor volume on the day of evaluation, and Vt2 represents the tumor volume on the day of grouping.

(Equation 3) $IR(\%) = [1-(RTVtest)/(RTVcont)] \times 100,$

wherein IR represents a tumor growth inhibition rate, RTVtest represents the average REV value in a drug administration group, and RTVcont represents the average RTV value in an untreated group.

[Table 5]

| Test Compound | IR(%) |
|---|---|
| Compound 1 | 96 |
| Compound 4 | 93 |
| Compound 7 | 96 |
| Compound 8 | 76 |
| Compound 9 | 78 |
| Compound 10 | 89 |
| Compound 11 | 93 |
| CNDAC | 54 |
| Compound 15 | 22 |
| Compound 16 | 38 |
| Compound 17 | 58 |

The results shown in Table 5 reveal that the compounds of the present invention (Compounds 1, 4, and 7 to 10) had higher antitumor effects than CNDAC, and that the antitumor effects of the compounds of the present invention were also higher than those of Compounds 15 to 17.

[Test Example 2]

Measurement of Water Solubility

Test compounds (2 mg or more) were each suspended in water (1 ml), then sonicated for 10 seconds, and incubated at 37 °C for 75 minutes. After filtration through a membrane filter (Millex-LG, 0.2 μm, product of Millipore Corporation), 100 μl of the obtained filtrate was diluted with acetonitrile (100 μl), and analyzed using a high performance liquid chromatograph apparatus. The solubility of each test compound in water was calculated from the calibration curve. The results are shown in Table 6.

[Test Example 3]

Pharmacokinetic Testing in SD (IGS) Rats

Test compounds were orally administered to SD (IGS) rats (product of Charles River Laboratories Japan, Inc.; 8 weeks old), and, based on the CNDAC serum concentration in the rats, orally administered absorbability and in vivo activation into CNDAC were evaluated.

In the morning of the test day, predetermined amounts of test compounds were each dissolved or suspended in a 0.5% hydroxypropylmethylcellulose solution prepared with 100mM citrate buffer (pH 4.5), and orally administered. CNDAC was administered at a dose of 10 mg/kg of body weight, and other test compounds were administered at a dose equivalent to 10 mg of CNDAC/kg of body weight. Blood was collected from the jugular vein 0.5, 1, 2, 4, 6, and 8 hours after administration of the test compounds (two or three rats per point) to obtain serum. The serum concentrations of the compounds and CNDAC were measured using LC/MS, and the area under the curve (AUC) of the CNDAC serum concentration from 0 to 8 hours after administration were calculated. The results are shown in Table 6.

[Table 6]

| Test Compound | Water Solubility (μg/ml) | AUC 0-8 hr (ng·hr/ml) |
|---|---|---|
| Compound 1 | 21.1 | 1136 |
| Compound 4 | 50 < | 1194 |
| Compound 7 | 50 < | 1073 |
| Compound 8 | 50 < | 836 |
| Compound 9 | 50 < | 921 |
| Compound 10 | 50 < | 926 |
| Compound 11 | 49.5 | 890 |
| CNDAC | 1000 < | 493 |
| P-CNDAC | < 0.5 | 956 |

As shown in Table 6, although CNDAC had extremely high water solubility, the AUC was as extremely low as 493 (ng·hr/ml). Further, P-CNDAC, which is a known oral CNDAC compound, had low water solubility. On the contrary, the compounds of the present invention (Compounds 1, 4, and 7 to 11) had excellent water solubility and high AUC.

[Test Example 4]

Antitumor Test of CNDAC, P-CNDAC, and Compounds 1 and 11 of the Present Invention using Tumor-Subcutaneously-Implanted Nude Mouse System upon Oral Administration at an Equitoxic Dose.

Human colon cancer strain KM20C subcutaneously subcultured in a BAZB/cA Jcl-nu mouse (product of Clea Japan Inc.) was prepared into fragments of 2 mm × 2mm, and subcutaneously implanted into the backs of 6-week-old BALB/cA Jcl-nu mice. On the 15th day after the day of implantation, the length of each tumor was measured along the major axis and minor axis, and the tumour volume was calculated using the following equation. The mice were then divided into several groups to reduce variations in tumor volume among the groups (5 or 6 mice per group).

$$\text{(Equation 5)} \quad Vt = 1/2(Vl) \times (Vs)^2$$

wherein Vt represents a tumor volume, Vl represents the length along the tumor major axis, and Vs represents the length along the tumor minor axis.

CNDAC, P-CNDAC, and Compounds 1 and 11 were each dissolved or suspended in a 0.5% hydroxypropylmethylcellulose solution prepared with 100 mM citrate buffer (pH 5.0), and, from the day after the day of grouping, orally administered at an equitoxic dose once per day for 14 days.

With respect to the mice in each group, the length of each of the subcutaneously implanted tumors was measured along the major axis and minor axis twice a week. RTV was calculated as a reference of tumor growth using the following equation to evaluate antitumor effects. The test results are shown in Figure 1.

$$\text{(Equation 6)} \quad RTV = Vt1/Vt2,$$

wherein RTV represents a relative tumor volume, Vt1 represents the tumor volume on the day of evaluation, and Vt2 represents the tumor volume on the day of grouping.

The results of Figure 1 show that, at an equtoxic dose, Compounds 1 and 11 achieved greater reduction of tumor volume than CNDAC and P-CNDAC. Further, in the case of CNDAC and P-CNDAC, tumor regrowth was observed on the $20^{th}$ to the $25^{th}$ day after the day of grouping (about 1 week after completion of administration). On the contrary, in the case of Compounds 1 and 11, the tumor size continued to reduce even after completion of administration. Moreover, although tumors did not disappear when treated with CNDAC or P-CNDAC, when treated with Compound 1, tumor disappearance was achieved in one of the five cases.

This accordingly reveals that the compounds of the present invention having excellent water solubility and high AUC are oral antirumor agents that are more effective than CNDAC and P-CNDAC.

Formulation Example 1: Tablets

[Table 7]

| | |
|---|---|
| Compound 11 | 50 mg |
| Corn starch | 50 mg |
| Microcrystalline cellulose | 50 mg |
| Hydroxypropyl cellulose | 15 mg |
| Lactose | 47 mg |
| Talc | 2 mg |
| Magnesium stearate | 2 mg |
| Ethyl cellulose | 30 mg |
| Unsaturated glyceride | 2 mg |
| Titanium dioxide | 2 mg |

According to the above formulation, tablets each weighing 250 mg were prepared by a standard method.

Formulation Example 2: Granules

[Table 8]

| | |
|---|---|
| Compound 1 | 300 mg |
| Lactose | 540 mg |
| Corn starch | 100 mg |
| Hydroxypropyl cellulose | 50 mg |
| Talc | 10 mg |

According to the above formulation, granules were prepared by a standard method, each dose weighing 1000 mg.

Formulation Example 3: Capsules

[Table 9]

| | |
|---|---|
| Compound 10 | 100 mg |
| Lactose | 30 mg |
| Corn starch | 50 mg |
| Microcrystal cellulose | 10 mg |
| Magnesium stearate | 3 mg |

According to the above formulation, capsules each weighing 193 mg were prepared by a standard method.

Formulation Example 4: Injection

[Table 10]

| | |
|---|---|
| Compound 4 | 100 mg |
| Sodium chloride | 3.5 mg |
| Distilled water for injection | Proper quantity (2 ml per ample) |

According to the above formulation, an injection was prepared by a standard method.

Formulation Example 5: Syrup

[Table 11]

| | |
|---|---|
| Compound 7 | 200 mg |
| Purified sucrose | 60 g |
| Ethyl p-hydroxybenzoate | 5 mg |
| Butyl p-hydroxybenzoate | 5 mg |
| Flavoring | Proper quantity |
| Colorant | Proper quantity |
| Purified water | Proper quantity |

According to the above formulation, a syrup was prepared by a standard method.

Formulation Example 6: Suppository

[Table 12]

| | |
|---|---|
| Compound 8 | 300 mg |
| Witepsol™ W-35 (mixture of mono-, di-, and triglycerides of saturated fatty acid from lauric acid to stearic acid; product of Dynamit Nobel AG) | 1400 mg |

According to the above formulation, a suppository was prepared by a standard method.

## Claims

**1.** A pyrimidine nucleoside compound represented by the following formula (1):

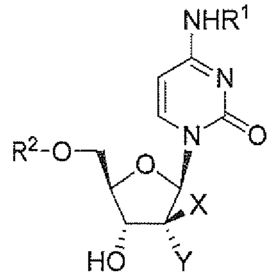

(1)

wherein one of X and Y is cyano and the other is hydrogen;
$R^1$ is hydrogen, alkyloxycarbonyl wherein the alkyl moiety is straight or branched $C_9$ alkyl, or alkenyloxycarbonyl wherein the alkenyl moiety is straight or branched $C_9$ alkenyl; and
$R^2$ is hydrogen or alkyloxycarbonyl wherein the alkyl moiety is straight or branched $C_{5-12}$ alkyl (the alkyl may be substituted), provided that:

i) when $R^1$ is hydrogen, $R^2$ is alkyloxycarbonyl wherein the alkyl moiety is straight or branched $C_{3-12}$ alkyl (the alkyl may be substituted), and
ii) when $R^1$ is alkyloxycarbonyl wherein the alkyl moiety is straight or branched $C_9$ alkyl or alkenyloxycarbonyl wherein the alkenyl moiety is straight or branched $C_9$ alkenyl, $R^2$ is hydrogen;

or a salt thereof.

**2.** A pyrimidine nucleoside compound or a salt thereof according to claim 1, wherein one of X and Y is cyano and the other is hydrogen;
$R^1$ is hydrogen; and
$R^2$ is alkyloxycarbonyl wherein the alkyl moiety is straight or branched non-substituted $C_{5-12}$ alkyl.

**3.** A pyrimidine nucleoside compound or a salt thereof according to claim 1 or 2, wherein one of X and Y is cyano and the other is hydrogen;
$R^1$ is hydrogen; and
$R^2$ is alkyloxycarbonyl wherein the alkyl moiety is straight non-substituted $C_{6-8}$ alkyl, or alkyloxycarbonyl wherein the alkyl moiety is branched non-substituted $C_9$ alkyl.

**4.** A pyrimidine nucleoside compound or a salt thereof according to any one of claims 1 to 3, wherein one of X and Y is cyano and the other is hydrogen;
$R^1$ is hydrogen; and
$R^2$ is n-hexyloxycarbonyl, n-heptyloxycarbonyl, n-octyloxy carbonyl, or nonan-5-yloxycarbonyl.

**5.** A pyrimidine nucleoside compound or a salt thereof according to claim 1, wherein one of X and Y is cyano and the other is hydrogen;
$R^2$ is hydrogen; and
$R^1$ is alkyloxycarbonyl wherein the alkyl moiety is straight or branched $C_9$ alkyl, or alkenyloxycarbonyl wherein the alkenyl moiety is straight $C_9$ alkenyl.

**6.** A pyrimidine nucleoside compound or a salt thereof according to claim 1 or 5, wherein one of X and Y is cyano and the other is hydrogen;
$R^2$ is hydrogen; and
$R^1$ is n-nonyloxycarbonyl, nonan--5-yloxycarbonyl, or alkenyloxycarbonyl wherein the alkenyl moiety is straight $C_9$ alkenyl having a stereochemistry of Z.

**7.** A pyrimidine nucleoside compound or a salt thereof according to claim 1, 5, or 6, wherein one of X and Y is cyano and the other is hydrogen;
$R^2$ is hydrogen; and
$R^1$ is n-nonyloxycarbonyl, (Z)-3-nonen-1-yloxycarbonyl, (Z)-6-nonen-1-yloxycarbonyl, or (Z)-2-nonen-1-yloxycarbonyl.

**8.** A pyrimidine nucleoside compound in any one of the following (a) to (n):

(a) 4-N-(n-nonyloxycarbonyl)-2'-cyano-2'-deoxy-1-β-D-arabinofuranosylcytosine,
(b) 4-N-(nonan-5-yloxycarbonyl)-2'-cyano-2'-deoxy-1-β-D-arabinofuranosylcytosine,
(c) 4-N-[(Z)-6-nonen-1-yloxycarbonyl]-2'-cyano-2'-deoxy-1-β-D-arabinofuranosylcytosine,
(d) 4-N-[(Z)-3-nonen-1-yloxycarbonyl]-2'-cyano-2'-deoxy-1-β-D-arabinofuranosylcytosine,
(e) 4-N-[(E)-2-nonen-1-yloxycarbonyl]-2'-cyano-2'-deoxy-1-β-D-arabinofuranosylcytosine,
(f) 4-N-(8-nonen-1-yloxycarbonyl)-2'-cyano-2'-deoxy-1-β-D-arabinofuranosylcytosine,
(g) 4-N-[(Z)-2-nonen-1-yloxycarbonyl]-2'-cyano-2'-deoxy-1-β-D-arabinofuranosylcytosine,
(h) 5'-O-(n-hexyloxycarbonyl)-2'-cyano-2'-deoxy-1-β-D-arabinofuranosylcytosine,
(i) 5'-O-(n-heptyloxycarbonyl)-2'-cyano-2'-deoxy-1-β-D-arabinofuranosylcytosine,
(j) 5'-O-(n-octyloxycarbonyl)-2'-cyano-2'-deoxy-1-β-D-arabinofuranosylcytosine,
(k) 5'-O-(nonan-5-yloxycarbonyl)-2'-cyano-2'-deoxy-1-β-D-arabinofuranosylcytosine,
(l) 5'-O-(n-nonyloxycarbonyl)-2'-cyano-2'-deoxy-1-β-D-arabinofuranosylcytosine,
(m) 5'-O-(heptan-4-yloxycarbonyl)-2'-cyano-2'-deoxy-1-β-D-arabinofuranosylcytosine, and
(n) 5'-O-(2,6-dimethylheptan-4-yloxycarbonyl)-2'-cyano-2'-deoxy-1-β-D-arabinofuranosylcytosine,

or a salt thereof.

**9.** A pharmaceutical composition comprising an effective amount of a pyrimidine nucleoside compound or a salt thereof according to any one of claims 1 to 8 and a pharmaceutical carrier.

**10.** An antitumor agent comprising an effective amount of a pyrimidine nucleoside compound or a salt thereof according to any one of claims 1 to 8 and a pharmaceutical carrier.

**11.** Use of a pyrimidine nucleoside compound or a salt thereof according to any one of claims 1 to 8 for producing a pharmaceutical composition.

**12.** Use of a pyrimidine nucleoside compound or a salt thereof according to any one of claims 1 to 8 for producing an antitumor agent.

**13.** A pyrimidine nucleoside compound or a salt thereof according to any one of claims 1 to 8 for use in the prevention and/or treatment of a disease.

**14.** A pyrimidine nucleoside compound or a salt thereof according to any one of claims 1 to 8 for use in the prevention and/or treatment of a tumor.

**15.** A method for preventing and/or treating a disease, the method comprising administering an effective amount of a pyrimidine nucleoside compound or a salt thereof according to any one of claims 1 to 8 to a patient.

**16.** A method for preventing and/or treating a tumor, the method comprising administering an effective amount of a pyrimidine nucleoside compound or a salt thereof according to any one of claims 1 to 8 to a patient.

[Figure 1]

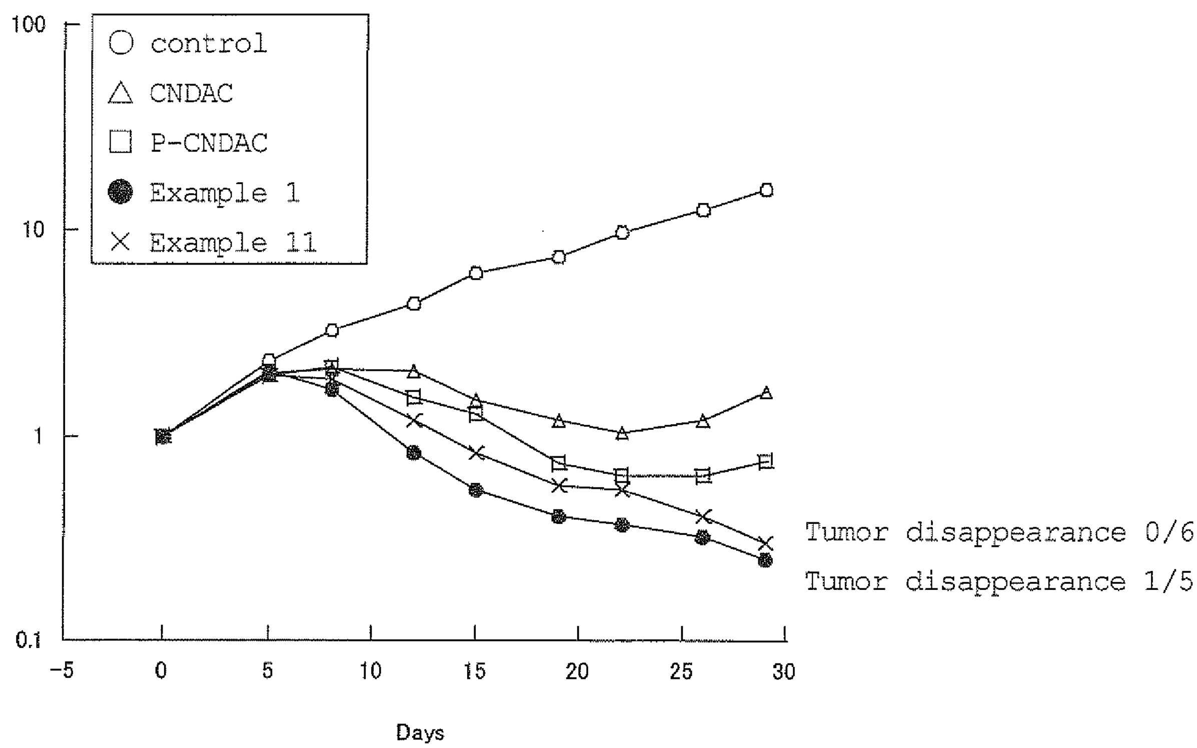
100
10
1
0.1
-5
0
5
10
15
20
25
30
Days
control
CNDAC
P-CNDAC
Example 1
Example 11
Tumor disappearance 0/6
Tumor disappearance 1/5

**INTERNATIONAL SEARCH REPORT**

International application No.
PCT/JP2007/064328

A. CLASSIFICATION OF SUBJECT MATTER
*C07H19/073*(2006.01)i, *A61K31/7068*(2006.01)i, *A61P35/00*(2006.01)i

According to International Patent Classification (IPC) or to both national classification and IPC

B. FIELDS SEARCHED

Minimum documentation searched (classification system followed by classification symbols)
C07H19/00-99/00, A61K31/00-31/80, A61P1/00-43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
WPI, CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 5-194497 A (Sankyo Co., Ltd.),<br>03 August, 1993 (03.08.93),<br>Claims; examples<br>& EP 536936 A1 & AU 9226026 A<br>& FI 9204381 A & HU 62310 T<br>& NO 9203794 A & CA 2079413 A<br>& TW 205042 A & ZA 9207474 A<br>& CZ 9202985 A3 & NZ 244574 A<br>& CN 1072686 A & AU 654212 B<br>& EP 536936 B1 & NO 179675 B<br>& DE 69212759 E & JP 2569251 B2<br>& ES 2093211 T3 & IL 103301 A<br>& US 5691319 A & RU 2085557 C1<br>& BR 1100621 A3 & FI 105556 B1<br>& KR 255491 B1 & CZ 289477 B6<br>& HU 221812 B1 & CA 2079413 C<br>& MX 9205623 A1 & PH 1199245022 B1 | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 August, 2007 (06.08.07) | 14 August, 2007 (14.08.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/064328

C (Continuation). DOCUMENTS CONSIDERED TO BE RELEVANT

| Category* | Citation of document, with indication, where appropriate, of the relevant passages | Relevant to claim No. |
|---|---|---|
| | & MX 221672 B | |
| Y | WO 2004/041203 A2 (XENOPORT INC.),<br>21 May, 2004 (21.05.04),<br>Claims; examples<br>& US 2004/142857 A1 & AU 2003291726 A1<br>& EP 1567169 A2 & AU 2003291726 A8 | 1-14 |
| P,X | WO 2006/080509 A1 (Taiho Pharmaceutical Co., Ltd.),<br>03 August, 2006 (03.08.06),<br>Full text<br>(Family: none) | 1-14 |
| A | JP 2004-505899 A (Koron Industries Inc.),<br>26 February, 2004 (26.02.04),<br>& WO 2002/011668 A2 & AU 200178804 A<br>& KR 2002012916 A & EP 1311524 A2<br>& KR 2003009649 A & US 2003/166606 A1<br>& CN 1446225 A | 1-14 |
| A | WO 91/19713 A1 (Sankyo Co., Ltd.),<br>26 December, 1991 (26.12.91),<br>& JP 04-235182 A & EP 535231 A1<br>& HU 62582 T & EP 535231 B1<br>& DE 69109482 E & ES 2074719 T3<br>& JP 2559917 B2 & US 5616567 A<br>& US 5654420 A & KR 9711306 B1<br>& RU 2116306 C1 & HU 218924 B<br>& CA 2085345 C | 1-14 |

Form PCT/ISA/210 (continuation of second sheet) (April 2005)

**INTERNATIONAL SEARCH REPORT**

International application No.
PCT/JP2007/064328

**Box No. II Observations where certain claims were found unsearchable (Continuation of item 2 of first sheet)**

This international search report has not been established in respect of certain claims under Article 17(2)(a) for the following reasons:

1. [X] Claims Nos.: 15, 16
because they relate to subject matter not required to be searched by this Authority, namely:
The inventions as set forth in claims 15 and 16 pertain to methods for treatment of the human body by therapy (PCT Article 17(2)(a)(i) and PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee..
[ ] The additional search fees were accompanied by the applicant's protest but the applicable protest fee was not paid within the time limit specified in the invitation.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H0637394 B
- JP 2559917 B
- JP 2569251 B
- JP H07179491 B
- JP 2005000204 B
- JP 6301483 W


### Non-patent literature cited in the description


- **EVANCE, J.S. et al.** *Proc. Soc. Exp. Bio. Med.,* 1961, vol. 106, 350
- **HOSHI, A. et al.** *Gann,* 1972, vol. 67, 725
- **KODAMA, K. et al.** *Jpn. J. Cancer Res.,* 1989, vol. 80, 679-685
- **MATSUDA, A. et al.** *Cancer Sci.,* 2004, vol. 95, 105-111
- **MATSUDA, A. et al.** *J. Med. Chem.,* 1991, vol. 34, 2919-2922
- **AZUMA, A. et al.** *J. Med. Chem.,* 1993, vol. 36, 4183-4189
- **MATSUDA, AKIRA ; SASAKI, TAKUMA.** *Tanpakushitsu Kakusan Kouso,* 1998, vol. 43, 1981-1989
- **KATZ, M.H. et al.** *Cancer Res.,* 2004, vol. 64, 1828-1833